# EUROPEAN PATENT APPLICATION

(11) **EP 1 889 639 A2**
(43) Date of publication of application: **20.02.2008**
(21) Application number: 07020539.8
(22) Date of filing: 07.09.2000
(51) Int. Cl.: A61P 35/00, A61K 31/135, A61K 31/235, A61K 31/33, A61K 31/519, A61K 31/4045, A61K 47/48

(54) **Methods and compositions containing succinimide or maleimide derivatives of antineoplastic agents, for producing long lasting antineoplastic agents**

(30) Priority: 07.09.1999 US 152681 P
(62) Divisional of application: 00962764.7
(71) Applicant: ConjuChem Biotechnologies Inc., Montreal, QC H2X 3Y8 (CA)
(72) Inventor: Bridon, Dominique, P., Ville Mont-Royal Quebec H3P 2S2 (CA); Leger, Roger, St-Lambert Quebec J4R 2V8 (CA); Huang, Xicai, Kirkland Quebec H9J 3X2 (CA); Milner, Peter, G., Los Altos Hills CA 94022 (US); Smith, Damon, Westmount Quebec H3Z 2E9 (CA); Ezrin, Alan, M., Moraga California 94556 (US)
(74) Representative: Williams, Gareth Owen

(57) **Abstract**

Derivatives of antineoplasfic agents are prepared. The derivatives are capable of forming covalent bonds with one or more blood components, preferably a mobile blood component.

## Description

### FIELD OF THE INVENTION

This invention relates to modified antineoplastic agents. In particular, this invention relates to antineoplastic agents that have been modified so that they are capable of forming a covalent bond with blood components *in vivo.*

### BACKGROUND OF THE INVENTION

Since the discovery of the antineoplastic properties of the nitrogen mustards more than 50 years ago, cancer chemotherapy has been an ever expanding area of scientific endeavor, and has been a critical component of cancer treatment along with surgery and radiation therapy. Where chemotherapy was once accepted only as a means to extend survival time for those patients diagnosed as incurable by surgery and/or radiation therapy, It is now a recognized modality of treatment in nearly all of the more than two thousand variations of cancer.

Modem cancer chemotherapy typically involves a combination of two or three different antineoplastic agents, and the advances in technology and medical knowledge have greatly improved a patient's chances of recovery in many forms of cancer. The role of antineoplastic agents in cancer therapy varies widely depending upon the form of cancer. For example, chemotherapy is often the primary course of therapy in cancers of the ovary, testis, breast, bladder, and others, in leukemias and lymphomas, and is generally employed in combination with radiation therapy in the treatment of a large number of sarcomas, melanomas, myelomas, and others. In contrast, chemotherapy is often used only as a last resort or as a palliative treatment for most solid tumors, such as carcinomas of the pancreas and lung. There are exceptions within each class of tumor or other neoplasm.

Native antineoplastic agents suffer from poor bio-availability, extensive first pass metabolism, and short half-lives, resulting in the need for these drugs to be constantly infused to the patient. In addition, while useful against cancer by killing cancer cells, antineoplastic agents are also often toxic to normal cells, a toxicity which is related to their uncontrolled biodistribution.

There is therefore a need for a method of prolonging the half-life of antineoplastic agents. At least two publications have addressed this problem. European Patent Application No. 0554708A1 to King discloses drugs modified with ligands attached to the drug with a hydrazone bond, as well as drugs attached to carriers through a hydrazone bond. In addition, PCT Application WO 98/10794 to Kratz discloses conjugates of transferrin, albumin and polyethylglycol with at least one HS, HO or H₂N group derived through maleimide or N-hydroxysuccinimide ester compounds.

The conjugates disclosed in these publications are made using methods that are practiced *ex vivo.* Such methods require obtaining human serum, purifying the desired carrier from the serum (for example albumin or transferrin), reacting the purified protein with the specific reactive groups (for example the "ligands" disclosed in the King application), and again purifying the conjugates to obtain a composition suitable for *In vivo* use. Such methods are quite invasive, lengthy and expensive. Therefore, there is a need to develop anti-cancer drugs with better bioavailability that can be introduced *In vivo* without requiring the step of conjugation *in vitro.*

### SUMMARY OF THE INVENTION

This invention relates to novel chemically reactive derivatives of antineoplastic agents, methods of synthesizing such derivatives, and methods of using such derivatives *in vivo.* The antineoplastic agent derivatives of the present invention can react *in vivo* to form covalent linkages with available functionalities on mobile blood proteins, such that the resulting covalently bound conjugates substantially retain their anti-cancer activity.

One aspect of the present invention is directed to the use of compositions in the manufacture of a medicament, the compositions including a derivative of an antineoplastic agent and analogs thereof. The derivative includes a reactive group which reacts with amino groups, hydroxyl groups, or thiol groups on blood components to form stable covalent bonds. The reactive group is selected from N-hydroxysuccinimide, *N*-hydroxy-sulfosuccinimide and a maleimide group for use In the treatment cancer. Specific examples of the antineoplastic agent used in the present invention include doxorubicins, taxols, methotrexates, hydroxyurea, tamoxifen, mytomycin C, and analogs thereof. Specific examples of a blood component with which the derivative is reactive include blood proteins, more specifically, a thiol group on a blood protein, and even more specifically, albumin.

Another aspect of the present invention is directed to methods for extending the lifetime of a antineoplastic agent *in vivo.* These methods include the steps of: modifying the antineoplastic agent by attaching a reactive group to a site sufficiently far away from the pharmacophore region such that the antineoplastic agent derivative substantially retains its therapeutic activity and is capable of forming a covalent bond with a functionality on a blood component; and forming a covalent bond between said reactive group and a functionality on a blood component to form a antineoplastic agent derivative-blood component conjugate, thereby extending the lifetime of the antineoplastic agent Specifically, the invention includes methods in which the step of administering the antineoplastic agent derivative *in vivo* occurs before the formation of the covalent bond, such that the antineoplastic agent derivative-blood component conjugate is formed *in vivo.*

Yet another aspect of the invention includes antineoplastic agent derivatives and methods of making such derivatives. The antineoplastic agent derivatives comprise an antineoplastic agent and a linking group together with a reactive group, for example maleimide or N-hydroxysuccinimide (NHS), capable of reacting with a functionality on a blood component. Specific antineoplastic agent derivatives include derivatives of doxorubicins, taxols, methotrexates, hydroxyurea, tamoxifen, mytomycin C, platinum complexes, vinca alkaloids, and analogs thereof.

By reacting with the blood component *in vivo* and conjugating thereto, the antineoplastic agent derivative of the invention may be delivered via the blood to appropriate sites or receptors. As compared with nonconjugated antineoplastic agents, the conjugated molecules have extended lifetimes in the bloodstream. Therefore, the conjugated antineoplastic agents are capable of maintaining anti-cancer activity for extended periods of time as compared to the non-conjugated parent drug, and provide such activity with reduced side effects.

The invention further includes conjugates of antineoplastic agent derivatives and mobile blood components. This includes the specific labeling of mobile endogenous proteins such as serum albumin, IgG, and proteins on interstitial and blood borne cells with antineoplastic agents derivatized with maleimide as the reactive group (maleimide-antineoplastic agents), as well as the non-specific labeling of mobile blood proteins with antineoplastic agents derivatized with NHS as the reactive group (NHS-antineoplastic agents) and sulfo-NHS as the reactive group (sulfo NHS-antineoplastic agents). The invention further includes methods for providing anti-cancer activity *in vivo* comprising administering to a mammalian host the novel antineoplastic agent derivatives or their conjugates.

This invention also relates to use of antibodies to locate and bind to such conjugates, for instance, to remove undesirable excesses of them from the host's blood stream. The invention also relates to the use of antibodies to detect levels of antineoplastic agent conjugates in blood,

Generally, an antineoplastic agent derivative according to the present invention is a compound of formula:

**A-X-R-D**

wherein:
A is an antineoplastic agent,
X is a sensitive functional group, for example (but not limited to) an ether, thioether, secondary or tertiary amine, secondary or tertiary amide, ester, thioester, imine, hydrazone, semicarbazone, acetal, heml-scetal, ketal, hemi-ketal, aminal, hemi-aminal, sulfonate, sulphate, sulfonamide, sulfonamidate, phosphate, phophoramide, phosphonate, or phosphonamidate;
R is an optional linking group, for example (but not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole; and
D is a reactive group, for example a carboxyl, phosphoryl, or acyl group, either as an ester or a mixed anhydride or an imidate, including phenolic esters, thiol esters, alkyl esters, phosphate esters, as well as succimidyl and maleimide groups, for example N-hydroxysuccinimide (NHS), N-hydroxy-sulfosuccinimide(sulfo-NHS), maleimide groups such as maleimide acids, (including but not limited to ma(eimidopropionicacid (MPA)) and maleimide esters.

It should be noted that usage of R as a linking group is optional. In some circumstances, it may be advantageous or even necessary to use a linking group In order to better retain the therapeutic activity of the antineoplastic agent. In other circumstances, however, the usage of a linking group may not be necessary, as the attachment of the reactive group (D) directly to the sensitive functional group (X) of the antineoplastic agent may sufficiently preserve the antineoplastic agent's therapeutic activity.

Specific antineoplastic agent derivatives of the present invention include doxorubicin derivatives such as a compound of formula: 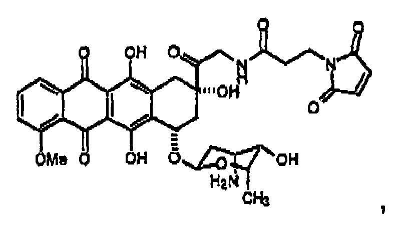 taxol derivatives such as a compound of formula: 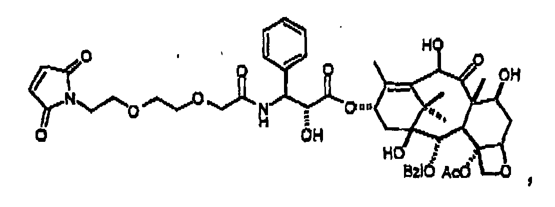 methotrexate derivatives such as compounds of formulae: 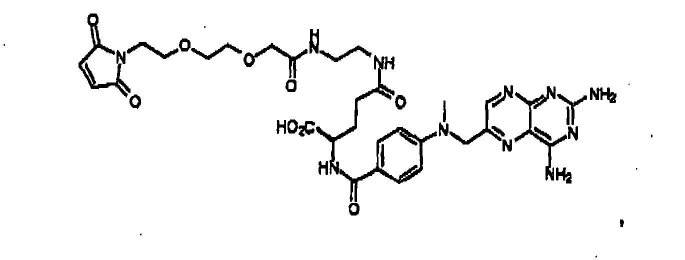 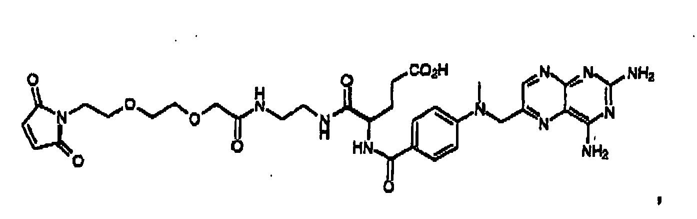 and 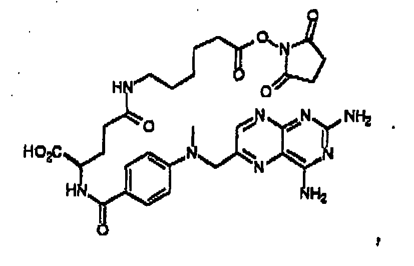 hydroxyurea derivatives such as a compound of formula: 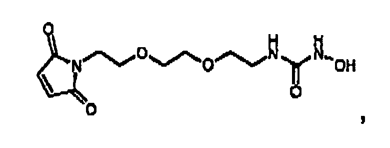 tamoxifen derivatives such as compounds of formulae: 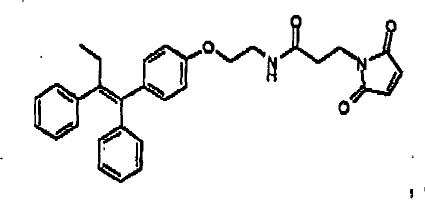 and 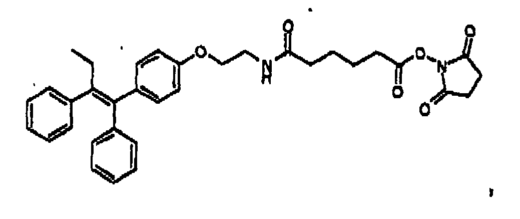 mytomycin C derivatives such as a compound of formula: 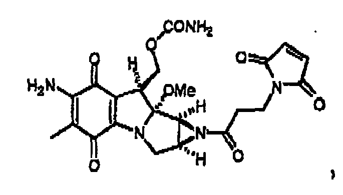 and
vincristine derivatives such as a compound of formula:

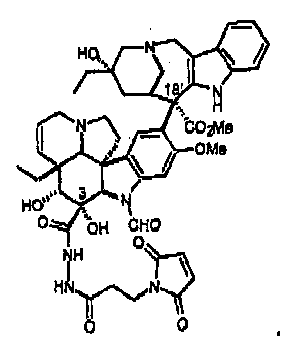

### DETAILED DESCRIPTION OF THE INVENTION

For the purpose of the present invention and to ensure a complete understanding of the invention the following definitions are provided:
**Antineoplastic Agents** - Antineoplastic agents are anti-cancer agents such as fluoropyrimidines, pyrimidine nucleosides, purines, platinum analogs, anthracyclines/anthracenedlones, podophyllotoxins, camptothecins, hormones and hormonal analogs, enzymes, proteins and antibodies, vinca alkaloids, taxanes or colchicine, atihormonal agents, antifolates, antimicrotubule agents, alkylating agents (classical and non-classical), antimetabolites, antibiotics, topoisomerase inhibitors, antivirals, and miscellaneous cytotoxic agents, for example hydroxyurea, mitotane, fusion toxins, PZA, bryostatin, retinoids, butyric acid and derivatives, pentosan, fumagillin, and others. The objective of all antineoplastic drugs is to eliminate (cure) or to retard the growth and spread (remission) of cancer cells. The majority of the above listed antineoplastic agents pursue this objective by possessing primary cytotoxic activity, effecting a direct kill on the cancer cells, Other antineoplastic drugs stimulate the body's natural immunity to effect cancer cell death.

The antineoplastic agents used in this invention may contain an asymmetric carbon atom or one or more asymmetric centers and may thus give rise to optical isomers and diastereomers. While shown without respect to stereochemistry, the antineoplastic agents used in the present invention Include such optical isomers and diastereomers; as well as the racemic and resolved, enantiomerically pure R and S stereoisomers; as well as other mixtures of the R and S stereoisomers and pharmaceutical acceptable salts thereof. Each antineoplastic agent includes a pharmacophore region that interacts with target sites, for example receptors, within the body.

**Reactive Groups:** Reactive groups are functional groups capable of forming a covalent bond. Such reactive groups are coupled or bonded to an antineoplastic agent of interest via a linking group (as defined below) and a hydrolytically stable bond, or optionally, without a linking group. Reactive groups will generally be stable in an aqueous environment and will usually be carboxyl, phosphoryl, or convenient acyl group, either as an ester or a mixed anhydride, or an imidate, thereby capable of forming a covalent bond with functionalities such as an amino group, a hydroxy or a thiol at the target site on blood components. For the most part, the asters will involve phenolic compounds, or be thiol esters, alkyl esters, phosphate eaters, or the like. Reactive groups include succimidyl and maleimide groups, for example N-hydroxysuccinimide (NHS), N-hydroxy-sulfosuccinimide (sulfo-NHS), maleimide groups such as maleimide acids, (including but not limited to maleimidopropionic acid (MPA)) and maleimide esters. In the preferred embodiments of this invention, the functionality on the blood component will be a thiol group and the reactive group will a maleimide group.

**Sensitive Functional Groups** - A sensitive functional group is a group of atoms that represents a potential reaction site on a therapeutic agent. If present, a sensitive functional group may be chosen as the attachment point for the linking group-reactive group modification. Sensitive functional groups include but are not limited to carboxyl, amino, thiol, hydroxyl, ether, thioether, secondary or tertiary amine, secondary or tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, or phosphonamidate;

**Functionalities:** Functionalities are groups on blood components including mobile proteins and fixed proteins, with which the reactive groups on the antineoplastic agent react to form covalent bonds. Functionalities usually include hydroxyl groups for bonding to ester reactive groups, thiol groups for bonding to maleimides, imidates and thioester groups; amino groups for bonding to activated carboxyl, phosphoryl or any other acyl groups on reactive groups.

**Blood Components:** Blood components may be either fixed or mobile. Fixed blood components are non-mobile blood components and include tissues, membrane receptors, interstitial proteins, fibrin proteins, collagens, platelets, endothelial cells, epithelial cells and their associated membrane and membraneous receptors, somatic body cells, skeletal and smooth muscle cells, neuronal components, osteocytes and osteoclasts and all body tissues especially those associated with the circulatory and lymphatic systems. Mobile blood components are blood components that do not have a fixed situs for any extended period of time, generally not exceeding 5, most often one minute. These blood components are not membrane-associated and are present in the blood for extended periods of time and are present in a minimum concentration of at least 0.1 µg/ml. Mobile blood components include serum albumin, transferrin, ferritin and immunoglobulins such as IgM and IgG. The half-life of mobile blood components is at least about 12 hours.

**Protecting Groups:** Protecting groups are chemical moieties utilized to protect the antineoplastic agent from reacting with itself. Specifically, the hydroxamic acid moiety of the antineoplastic agents used in the present invention is protected using protecting groups including but not limited to t-butyl, allyl, benzyl, tetrahydropyranyl, t-butyioxycarbonyl, t-butyldimethylsilyl and trimethylsilyl.

**Linking Groups:** Linking groups are chemical moieties that link or connect reactive groups to antineoplastic agents. Linking groups may comprise one or more alkyl groups, fluoroaklyl groups, alkoxy groups, alkenyl groups, alkynyl groups or amino groups substituted by alkyl groups, cycloalkyl groups, polycyclic groups, aryl groups, polyaryl groups, substituted aryl groups, heterocyclic groups, and substituted heterocyclic groups. Linking groups may also comprise poly ethoxy amino acids, such as AEA ((2-amino) ethoxy acetic acid) or a preferred linking group AEEA ([2-(2-amino)ethoxy)] ethoxy acetic acid.

**Antineoplastic Agent Derivatives** - An antineoplastic agent derivative is an antineoplastic agent that has been modified by attaching a reactive group. The reactive group may be attached to the antineoplastic agent via a linking group, or optionally without using a linking group. Usually, the antineoplastic agent derivative comprises the antineoplastic agent molecule and a linking group together with a reactive group capable of reaction with a functionality on a blood component. Antineoplastic agent derivatives may be administered *In vivo* such that conjugation with blood components occurs *in vivo,* or they may be first conjugated to blood components *in vitro* and the resulting conjugated antineoplastic agents (as defined below) administered *in vivo.*

**Conjugated Antineoplastic Agents** - A conjuged antineoplastic agent is an antineoplastic agent derivative that has been conjugated to a blood component via a covalent bond formed between the reactive group of the antineoplastic agent derivative (attached to the antineoplastic agent either with or without a linking group), and the functionality on the blood component. As used throughout this application, the term "conjugated antineoplastic agent" can be made more specific to refer to particular conjugated antineoplastic agents, for example "conjugated doxorubicin" or "conjugated taxol." By conjugating with the blood component, the antineoplastic agent derivative may be delivered via the blood to appropriate sites or receptors of the patient.

Taking into account these definitions, this invention relates to compositions which are derivatives of antineoplastic agents which can react with the available reactive functionalities on blood components via covalent linkages. The invention also relates to such derivatives, such combinations with blood components, such methods of making the derivatives, and methods for their use. These methods include extending the effective therapeutic life of the antineoplastic agent in question as compared to administration of the antineoplastic agent per se to a patient.

To form covalent bonds with the functionality on the blood component, one may use as a reactive group a wide variety of active carboxyl groups, particularly esters, where the hydroxyl moiety is physiologically acceptable at the levels required. While a number of different hydroxyl groups may be employed, the most convenient would be N-hydroxysucxinimide (NHS), N-hydraxy-sulfosuccinimide (sulfo-NHS), maleimide, maleimide acids including but not limited to maleimidopropionic acid (MPA), and maleimide esters. In the preferred embodiments of this invention, the functionality on the blood component will be a thiol group and the reactive group will a maleimide.

The aim of the present invention is to derivatize antineoplastic agents to confer to these inhibitors improved bio-avallability, extended half-life and better distribution through selective, conjugation of the inhibitor onto a protein carrier but without modifying their remarkable anti-cancer properties. The preferred carrier for this invention is, but not limited to, albumin, which is then conjugated through its free thiol by antineoplastic agent derivatized with a maleimide moiety.

### 1. Antineoplastic Agents Used in the Present Invention

Currently, there are approximately twenty recognized classes of approved antineoplastic drugs. The classifications are generalizations based on either a common structure shared by particular drugs, or are based on a common mechanism of action by the drugs. Although some drugs fall into two or more classes, in general, the accepted classifications are as follows (the classifications are listed in no particular order): fluoropyrimidines, pyrimidine nucleosides, purines, platinum analogs, anthracyclines/anthracenediones, podophyllotoxins, camptothecins, hormones and hormonal analogues, enzymes, proteins and antibodies, vinca alkaloids, taxanes, atihormonal agents, antifolates, antimicrotubule agents, alkylating agents (classical and non-classical), antimetabolites, antibiotics, topolsomerase inhibitors, antivirals, and miscellaneous cytotoxic agents as enumerated in more detail below.

A partial listing of some of the commonly known commercially approved (or in active development) antineoplastic agents organized by classification is as follows:
pyrimidine analogs such as fluoropyrimidines, for example 5-FU, fluorodeoxyuridine, ftorafur, 5'-deoxyfluorouridine. UFT, S-1 and capecitabine;
pyrimidine nucleosides such as deoxycytidine, cytosine arabinoside, 5-azacytosine, 9-beta-D-arabinofuranosyl, gemcitabine, and 5-azacytosine-arabinoside;
purines and purine analogs such as 6-mercaptopurine; thioguanine, azathioprine, allopurinol, cladribine, fludarabine, pentostatin, and 2-chloro adenosine;
platinum analogues and platnum coordination complexes such as cisplatin, carboplatin, oxaliplatin, tetraplatin, platinum-DACH, ormaplatin, Cl-973, and JM-216;
anthracyclines or anthracenediones such as doxorubicin, daunorubicin, epirubicin, idarubicin, and mitoxantrone;
epipodophyllotoxins such as etoposide and teniposide;
camptothecins such as irinotecan, topotecan, 9-amino camptothecin, 10,11-methylenedioxy camptothecin, 9-nitro camptothecin, and TAS 103:
hormones and hormonal analogs such as diethylstilbestrol, tamoxifen, toremefine, tolmudex, thymitaq, flutamide, bicalutamide, finasteride, estradiol, trioxifene, droloxifene, medroxyprogesterone acetate, megesterol acetate, aminoglutethimide, testolactone and others;
enzymes, proteins and antibodies such as asparaginase, interteukins, interferons, leuprolide, pegaspargase, and others;
vinca alkaloids such as vincristine, vinblastine, vinorelbine, vindesine;
taxanes such as paclitaxel and docetaxel, and colchicine;
antihormonals such as anastrozole;
antifolates such as methotrexate (amethopterin), aminopterin, trimetrexate, trimethoprim, pyritrexim, pyrimethamine, edatrexate, and MDAM;
antimicrotubule agents such as taxanes and vinca alkaloids;
alkylating agents (classical and non-classical) such as nitrogen mustards (mechlorethamine, chlorambucil, melphalan, uracil mustard), oxazaphosphorines (ifosfamide, cyclophosphamide, perfosfamide, trophosphamide), alkylsulfonates (busulfan), nitrosoureas (carmustine, lomustine, streptozocin), thiotepa, dacarbazine and others;
antimetabolites such as purines, pyrimidines and nucleosides, listed above;
antibiotics such as anthracyclines/anthracenediones, bleomycin, dactinomycin, mitomycin, plicamycin, pentostatin, streptozocin, daunorubidin (rubimycin), and doxorubicin;
topoisomerase inhibitors such as camptothecins (Topo I) epipodophyllotoxins, m-AMSA, and ellipticines (Topo II);
antivirals such as AZT, zalcitabine, gemcitabine, didanosine, and others; and
miscellaneous cytotoxic agents such as hydroxyurea, mitotane, fusion Toxins, PZA, bryostatin, retinoids, butyric acid and derivatives, pentosan, fumagillin, and others.

The primary difficulty and cause for concern with any antineoplastic drug is its toxicity to normal, healthy cells. All of the above listed drugs (and those drugs currently under development) have the potential to mediate serious and often life-threatening toxic side effects even when given in therapeutically effective dosages. Although extensive efforts have been made to develop antineoplastic drugs which are safe to use in effective doses, there are nearly always toxic side effects associated with such drugs. Specific manifestations of toxic side effects are available in any of a number of oncology textbooks, publications, patents, and other printed matter. Since the compounds of this invention have greater bioavailability and thus need not be administered in large quantities, they have better safety profiles than the non-derivatized antineoplastic agent.

### 2. Mechanisms of Action

Detailed mechanisms of action and toxicity, both proven and postulated, are recited in detail throughout the prior art. An overview of these mechanisms is set forth below to outline the metabolism of the antineoplastic drugs, and their effects on malignant, as well as on healthy, tissue.

### A. Antihormonals

As their name suggests, the antihormonal antineoplastic drugs exert cytotoxic activity by blocking hormone action at the end-receptor organ. Several different types of neoplasms require hormonal stimulation to propagate cell reproduction. The antihormonal agents, by blocking hormone action, deprive the neoplastic cells of a necessary stimulus to reproduce. As the cells reach the end of their life cycle, they die normally, without dividing and producing additional malignant cells.

Antihormonal drugs have been used with varying degrees of success as treatments for malignancies of the breast, uterus, prostate, testis, and other gender specific organs. Antihormonal drugs generally target androgen receptors in men, and estrogen or progesterone receptors in women. Recently, much research has been devoted to the development of aromatase inhibitors, which indirectly lower estrogen blood levels by blocking the conversion of androgens to estrogens. Antihormonal antineoplastic agents are essentially classified as steroidal or non-steroidal.

Toxicity of antihormonal antineoplastic agents is generally mild to moderate. Since the agents are usually close analogues of natural physiologic agents, they are relatively specific in their mechanism of action, and do not interfere with DNA synthesis, which minimizes the severe side effects seen in most all antineoplastic agents.

Manifestations of toxicity in antihormonal therapy include those effects normally associated with hormone deficiencies, such as hot flashes, weight gain (and weight loss), skin rashes, acne, and others. Nausea and vomiting may occur occasionally, but GI distress is normally mild to moderate and does not normally require dose reduction. Antihormonal therapy is normally limited to patients who exhibit positive responses to hormones.

### B. Antifolates

Antifolates exert cytotoxic activity by inhibiting the action of dihydrofolate reductase (DHFR), a critical enzyme in cell reproduction. It is well known that DHFR reduces dihydrofolates to their active, fully reduced tetrahydro form. The tetrahydrofolates act as coenzymes in the de novo synthesis of purines, which are critical precursors to DNA. Depletion of tetrahydrofolate pools also inhibits thymidylate biosynthesis, which blocks the conversion of deoxyuridine phosphate (dUMP) to deoxythymidine phosphate (dTMP), a DNA nucleotide precursor.

Although a number of antifolate drugs have been approved for use in the United States, only one, Methotrexate, is a commercially approved antineoplastic agent. The other approved antifolates are used primarily as anti-infective drugs, due to their propensity to bind to bacterial DHFR with much greater strength and frequency than human DHFR. Methotrexate itself has also been used in the treatment of rheumatoid arthritis, psoriasis, and related inflammatory type diseases, as well as against cancers of the breast, uterus, testis, ovary, and against various forms of leukemia (for example acute lymphocytic leukemia). Single agent Methotrexate is curative against choriocarcinoma, a rare type of cancer.

Antifolates produce a wide range of assorted toxic and other untoward effects. Hematologic toxicity is a major dose-limiting toxicity associated with methotrexate and other antifolates. Other toxic and untoward effects associated with antifolates include neurotoxicity, which is sometimes severe and irreversible; gastrointestinal toxicities, mainly mucositis, nausea, diarrhea, vomiting, anorexia, and others; hepatic toxicity; dermatologic toxicity, often manifested by rashes, alopecia, and tissue inflammation; pulmonary toxicity; renal toxicity; and other untoward toxic side effects, Antifolates are generally recognized as drugs with low therapeutic indices and high incidences of toxic side effects.

### C. Antimicrotubule Agents

Antimicrotubule agents interfere with cell division by disrupting the normal functionality of the cellular microtubules. Microtubules are critical elements of the cell, playing an important role in separation of the duplicate chromosomes during cell division, as well as being responsible for many interphase cellular functions, such as maintenance of cell shape and scaffolding, intracellular transport, secretion, neurotransmission, and others.

The two classes of antineoplastic agents which influence microtubules are the Taxanes (Paclitaxel and Docetaxel are the representative agents of this class) and the Vinca Alkaloids (vincristine, Vinblastine and Vinorelbine are approved members of this class),

Taxanes and Vinca Alkaloids are naturally or semisynthetically derived analogues of naturally occurring compounds derived from plants. In particular, Taxanes are derived from the needles and twigs of the European yew (*Taxus baccata*), or the bark of the Pacific yew (*Taxus brevifolia*). The most widely known Taxanes at this time are paclitaxel (Taxol^{®}) and docetaxel (Taxotere^{®}), which are widely marketed as antineoplastic agents.

Vinca Alkaloids include Vincristine (Oncovin^{®}), Vinblastine (Velban^{®}) and Vinorelbine (Navelbine^{®}), and are derived generally from the periwinkle plant (*Cantharanthus roseus*). These drugs are also widely marketed as antineoplastic drugs.

Although their mechanisms of action are different, both Taxanes and Vinca Alkaloids exert their biological effects on the cell microtubules. Taxanes act to promote the polymerization of tubulin, a protein subunit of spindle microtubules. The end result is the inhibition of depolymerization of the microtubules, which causes the formation of stable and nonfunctional microtubules. This disrupts the dynamic equilibrium within the microtubule system, and arrests the cell cycle in the late G₂ and M phases, which inhibits cell replication.

Like Taxanes, Vinca Alkaloids also act to affect the microtubule system within the cells. In contrast to Taxanes, Vinca Alkaloids bind to tubulin and inhibit or prevent the polymerization of tubulin subunits into microtubules. Vinca Alkaloids also induce the depolymerization of microtubules, which inhibits microtubule assembly and mediates cellular metaphase arrest. Vinca Alkaloids also exert effects on nucleic acid and protein synthesis; amino acid, cyclic AMP, and glutathione synthesis; cellular respiration; and exert immunosuppressive activity at higher concentrations.

Both Taxanes and Vinca Alkaloids are toxic compounds having a low therapeutic index. Neurotoxicity and myelosuppression are among the most commonly reported clinical toxicities of these drugs.

Taxanes have been shown to cause a number of different toxic and untoward side effects in patients. The most well-known and severe adverse effects of Taxanes are neurotoxicity and hematologic toxicity, particularly severe neutropenia and thrombocytopenia. Taxanes also cause hypersensitivity reactions in a large percentage of patients; GI effects (nausea, diarrhea and vomiting are common); alopecia; and other untoward effects at the recommended dosages.

Vinca Alkaloids have been associated primarily with neurotoxic effects (the primary adverse effect in patients receding Vincristine): hematologic toxicity (leukopenia is often severe and dose-limiting in the case of Vinblastine); gastrointestinal effects (nausea, diarrhea and vomiting); alopecia; tissue irritation and necrosis, particularly at the injection site; and other harmful, untoward effects.

### D. Alkylating Agents

As the name implies, alkylating agents generally exert cytotoxic activity by alkylating DNA, directly interfering with the reproductive cycle of the cell. As noted in the general listing above, antineoplastic alkylating agents fall into numerous subclasses based on the general structure and mechanism by which they act to alkylate DNA.

Several subclasses of antineoplastic alkylating agents are marketed as chemotherapeutic agents. The most well-known are the nitrogen mustards, which include Chlorambucil, Mechlorethamine, Melphalan, Uracil Mustard, and others.

Oxazaphosphorines are a subtype of the nitrogen mustards and include Ifosfamide, Cyclophosphamide, Trophosphamide and others. Other types of antineoplastic drugs which act as alkylating agents, and which are commercially available as of the date of this application include alkylsulfonates (Busulfan) and nitrosoureas (Carmustine, Lomustine and, to some extent, Streptozocin). Other antineoplastic drugs also possess the ability to alkylate DNA and/or RNA. Dacarbazine and Procarbazine are currently approved drugs in the United States which act as alkylators of DNA.

Antineoplastic alkylating agents produce a wide range of assorted toxic and other untoward side effects. Hematologic toxicity is a major dose-limiting toxicity associated with all alkylating agents, and is often severe. Other toxic and untoward effects associated with alkylating agents include neurotoxicity; gastrointestinal toxicities, mainly nausea, diarrhea, vomiting, anorexia, and others; hepatic toxicity; dermatologic and local area sensitivity, often manifested by rashes, alopecia, and tissue inflammation and necrosis; pulmonary effects; genitourinary effects (hemorrhagic cystitis is often severe in patients receiving cyclophosphamide therapy); and other untoward toxic side effects. Antineoplastic alkylating agents are universally recognized as drugs with low therapeutic indices and high incidences of toxic side effects.

### E. Antimetabolites

Antimetabolites generally exert cytotoxic activity by substituting fraudulent nucleotides into cellular DNA, thereby disrupting cellular division, or by inhibition of critical cellular enzymes, which prevents replication of DNA. Antimetabolites are necessarily comprised of purine (guanine or adenosine) or pyrimidine (cytidine or thymidine) bases, with or without the attached sugar moiety.

Purine antimetabolites are widely used antileukemic drugs, particularly in the treatment of various forms of childhood leukemia. 6-Mercaptopurine (6-MP) has been administered as a treatment for acute lymphoblastic leukemia (ALL) since the mid-1940s. 6-Thloguanine (6-TG) is currently used as a remission inducer in acute myelogenous leukemia (AML). Azathioprine, which is a prodrug of 6-MP, is widely used as an immunosuppressant agent in clinical transplantation. In general, purine antimetabolites are not effective against solid tumors, probably due to the extended lifespan of solid tumor cells compared to leukocytes.

Like other cytotoxic agents, purine antimetabolites also affect the function of normal, healthy cells. The cells most affected by the purine antimetabolites are those rapidly proliferating cells, which have relatively short lifespans, namely the cells found in bone marrow and the upper GI tract.

Purine antimetabolites generally produce a narrow range of toxic and other untoward effects. Hematologic toxicity is a major dose-limiting toxicity associated with 6-mercapto purine, Azathioprine, 6-thioguanine, and other purine antimetabolites. In particular, leukopenia, anemia, and thrombocytopenia have been widely reported effects of these drugs. Other toxic and untoward effects associated with purine antimetabolites include gastrointestinal toxicities, mainly nausea, diarrhea and vomiting; hepatic toxicity; and other untoward toxic side effects. Purine antimetabolites are generally recognized as drugs with low therapeutic indices and high incidences of toxic side effects.

In general, the nucleoside analogues all undergo phosphorylation, in vivo, usually by deoxycytidine kinase to their corresponding nucleotides. In this active form, the drugs are all potent antimetabolites, and are postulated to inhibit one or more enzymes necessary for DNA replication. Many of the antineoplastic nucleosides are cell-cycle specific, acting in the S phase, and perhaps at the G,-S border. Some of the enzymes which the antineoplastic antimetabolites are thought to inhibit include ribonucleotide reductase, DNA polymerase, thymidylate synthetase, uracil riboside phosphorylase, DNA primase, and others. Because of their inhibition of cellular replication enzymes, many of the antineoplastic nucleosides also exhibit antiviral activity.

Nucleoside antimetabolites produce a wide range of assorted toxic and other untoward effects. Hematologic toxicity is a major dose-limiting toxicity associated with all antineoplastic nucleosides, and is often severe. Other toxic and untoward effects associated with nucleosides Include neurotoxicity, which is sometimes severe and irreversible; gastrointestinal toxicities, mainly nausea, diarrhea, vomiting, anorexia, and others; hepatic toxicity: dermatologic toxicity, often manifested by rashes, alopecia, and tissue inflammation; pulmonary toxicity; and other untoward toxic side effects. Antineoplastic nucleosides are generally recognized as drugs with low therapeutic indices and high incidences of toxic side effects.

### F. Anthracyclines/Anthracenediones

The antineoplastic drugs of this class also possess antibiotic activity, and were originally developed for such purposes. Although the primary mechanism of action of these compounds is uncertain, it has been postulated that they act by inhibiting Topoisomerase II (Topo II) by the formation of cleavable complexes. Other mechanisms are also postulated to account for the wide spectrum of activity of the anthracyclines.

Because of their broad spectrum of activity, anthracyclines and anthracenedionediones have been extensively studied and reported on throughout the last 25 years. There are currently 3 of these agents approved for use in the US (Doxorubicin, Daunorubicin and Mitoxantrone), and two more (Epirubicin and Idarubicin) are approved for use in Europe.

Like other cytotoxic agents, antineoplastic anthracyclines and anthracen6diones also affect the function of normal, healthy cells. In particular, the use of these agents has been associated with cardiac toxicity of a type not observed with other antineoplastic agents. Cardiac toxicity is not common (about 20% experience significant problems), but may become chronic and life threatening. Congestive heart failure and other equally severe cardiac diseases occur occasionally in patients receiving anthracycline chemotherapy. In most cases, the adverse cardiac effects are associated with cumulative doses, and in some cases, the effects are irreversible.

Antineoplastic anthracyclines and anthracenediones can generally produce other toxic and untoward effects. Hematologic toxicity is a major dose-limiting toxicity associated with all antineoplastic anthracyclines and anthracenediones. In particular, leukopenia, anemia, and thrombocytopenia have been widely reported effects of these drugs. Other toxic and untoward effects associated with antineoplastic anthracyclines and anthracenediones include gastrointestinal toxicities, mainly mucositis, with nausea, diarrhea and vomiting also frequent; alopecia, which occurs in nearly all patients; severe local tissue damage after extravasation; and other untoward toxic side effects. Antineoplastic anthracyclines and anthracenediones, although having a very broad spectrum of activity, are generally recognized as drugs with low therapeutic indices and high incidences of toxic side effects.

Previous means of combating the toxic effects of the anthracyclines and anthracenediones were nonexistent. Dose reduction or discontinuance of the drug was necessary, adversely affecting the probability of remission or cure. Recently, an experimental drug, ICRF 187, which metabolizes to form a diamide analog of EDTA, has shown some efficacy in ameliorating the cumulative cardiac toxicity of the anthracyclines and anthracenediones.

### G. Other Antibiotics

Antineoplastic antibiotics exert their cytotoxic activity through diverse mechanisms. Pentostatin is a purine analogue and acts as an antimetabolite of that class. Bleomycin is a glycopeptide with a molecular weight on nearly 1500 Daltons, and oxidatively cleaves DNA. Actinomycin (also known as Dactinomycin) inhibits RNA and protein synthesis. Plicamycin (Mithramycin) binds to DNA, and Mitomycin and Streptozocin are DNA alkylating agents.

Toxicities associated with antibiotics are similar to other agents with similar mechanisms of action. Hematologic toxicity is quite common with these drugs and is at times severe and dose-limiting. Most of theses drugs are also extremely lrritating to tissues, so local extravasation is a concern. In addition, Mithramycin has been associated with a thrombocytopenia induced hemorrhagic syndrome, particularly with prolonged use or high doses of the drug.

Antineoplastic antibiotics are one of the oldest classes of oncology drugs (Actinomycin was introduced into the clinic in 1954), and are still widely used in the treatment of pediatric neoplasms (Actinomycin), testicular neoplasms (Bleomycin), and any of a number of other solid tumors.

### H. Topoisomerase Inhibitors

Topoisomerase inhibitors are relatively newly introduced antineoplastic agents, with many more agents currently in various stages of research and development. As more information is developed concerning the Topoisomerases, more agents which inhibit these enzymes will no doubt be studied.

Topoisomerases (hereinafter referred to as Topo I and Topo II) are ubiquitous cellular enzymes which initiate transient DNA strand breaks during replication to allow for free rotation of the strands. The functionality of these enzymes is critical to the replication process of DNA. Without them, the torsional strain in the DNA helix prohibits free rotation, the DNA strands Care unable to separate properly, and the cell eventually dies without dividing. Topo I links to the 3'-terminus of a DNA single strand break, while Topo II links to the 5'-terminus of a double strand DNA break.

### Epipodopyllotoxins

Epipodophyllotoxins inhibit the action of Topo II. Natural podophyllotoxin is derived from the mayapple and mandrake plants, and extracts of those plants were a source of folk medicine for several centuries. The discovery of antineoplastic properties of both naturally occurring podophyllotoxin and the semi-synthetic epipodophyllotoxins dates back more than 30 years.

Naturally occurring podophyllotoxin has no glucoside ring, and the C4 position on the E-ring is a methoxy moiety. Although the mechanism of action is not fully understood, it has been postulated that the E-ring hydroxy is responsible for inducing double strand DNA breaks by inhibiting the Topo II. while the glucoside moiety is thought to prevent the synthetic drug derivatives from interfering with spindle microtubule assembly.

Etoposide (and Etoposide Phosphate, which is a water soluble prodrug of Etoposide) is currently used in therapy for a variety of cancers, including testicular neoplasms, lung cancers, lymphomas, neuroblastoma, AIDS related Kaposi's Sarcoma, Wilms' Tumor, various types of leukemia, and others. Teniposide is a relatively new drug which was approved by FDA and launched commercially in the United States in 1992 as therapy for refractory childhood leukemia. Teniposide has also exhibited activity against bladder cancer, lymphomas, neuroblastoma, small cell lung cancer, and certain CNS tumors, although it is not approved for such uses.

Both Etoposide and Teniposide are poorly water soluble (less than 10 .mu.g/mL), and must be formulated with organic solvents for practical IV delivery. Etoposide is also available in an oral dosage form, as a liquid filled capsule.

The primary dose-limiting toxicity of the epipodophyllotoxins is neutropenia, which is often severe, particularly among patients who have been treated with other antineoplastic agents or radiation. Other hematologic toxicities are commonly reported, as are alopecia, GI distress, neurological toxicity, sensitivity reactions, and others. Further, some patients receiving etoposide have suffered congestive heart failure and/or myocardial infarction, which has been attributed many times to the large volume of saline diluent (and In some cases, the speed of the delivery) used to deliver the drug.

Podophyllotoxins exhibit complex pharmacokinetic properties. Etoposide decays into at least 6 identified metabolites, in vivo. It is known that at least two of these metabolites, the glucuronide and the transhydroxyacid metabolites possess little cytotoxic activity, and have been postulated to be responsible for the hematologic toxicity of the drug. Since etoposide phosphate is rapidly converted to etoposide, the same metabolites, toxicities, and pharmacokinetic properties are also attributed to the prodrug. Teniposide has not been studied as extensively as etoposide, but is presumed to have similar properties.

### Camptothecins

Although Camptothecins have been known for more than 30 years, their usefulness as antineoplastic agents has only been discovered recently. The gap between discovery and introduction is due mainly to the very poor water solubility exhibited by these compounds. For almost two decades after discovery of the active compound, research was directed towards the discovery of more highly water soluble camptothecins, since administration would be facilitated.

Researchers formulated the lipophilic drugs with sodium hydroxide in an effort to increase the water solubility of the drugs for administration. Unfortunately, these formulations were essentially inactive and also very toxic in vivo. It was discovered in the mid-1980s that in basic pH conditions, camptothecin derivatives hydrolyzed, with the E-ring lactone opening to form a carboxylate anion. This form of the compounds was discovered to have only one-tenth or less the antineoplastic activity of the lactone form, and was also discovered to be highly toxic to healthy cells.

Much research has been devoted since this discovery to developing water soluble camptothecin derivatives which remained In their active lactone form. Along these lines, the recently approved Irinotecan (CPT-11) and Topotecan were developed. Irinotecan is a water soluble prodrug of the highly active, highly lipophilic derivative of CPT known as SN38 (10-hydroxy-7-ethyl CPT). Topotecan incorporates a 9-dimethylaminoethyl moiety of the 10-hydroxy derivative of CPT.

Like many other antineoplastic agents, the primary toxicities of CPT derivatives are hematologic toxicity and Gl distress. Irinatecan has been associated with severe diarrhea, which is often dose limiting, as well as neutropenia, mucositis, other GI distress, alopecia and elevated liver function. Toxicities of Topotecan are similar, although the diarrhea is not normally severe.

Camptothecins are generally recognized as drugs with low therapeutic indices and high incidences of toxic side effects. Coadministration of a protective agent which reduces the toxic side effects of camptothecins will provide for a safer and more effective chemotherapeutic regimen. This may even allow higher doses of the antineoplastic drug to be given, thereby increasing the probability of success of treatment.

### 1. Platinum Complexes

Since the discovery of their antineoplastic properties more than 30 years ago, platinum complexes have been developed as therapeutic agents for many different types of solid tumors. Two such complexes, cisplatin and carboplatin, are in widespread use today, both as single agents and in combination therapy for tumors of the testis, ovary, lung, bladder, and other organs.

The mechanism of action of platinum complexes has been widely studied. Platinum complexes have been discovered to bind covalently to DNA, thereby disrupting DNA function, effecting direct cell kills. Platinum complexes also freely bind to proteins, and it is postulated that protein-bound platinum my also affect DNA.

Toxicity manifestations of cisplatin are entirely different than the toxicity of carboplatin. Cumulative, dose-limiting renal toxicity is common in cisplatin therapy, while hematologic toxicity similar to electrophilic alkylating agents is the major toxicity associated with carboplatin. Both cisplatin and carboplatin have also been associated with gastrointestinal distress, mainly nausea and vomiting, as well as neurotoxic effects.

Since platinum complexes are not extensively metabolized, in vivo, the platinum species present in the body depend upon the reactivity of the complex with water to form hydroxylated and aquated complexes. Further, platinum complexes are largely eliminated from the body through renal excretion, and the acidic conditions present in the kidneys tend to favor the formation of these generally Inactive (against neoplasms) and toxic species. In particular, the chloride atoms of cisplatin are readily displaced by hydroxy and aquo moieties under acidic conditions, accounting for the often severe renal toxicity associated with the drug.

Both cisplatin and carboplatin are highly lipophilic compounds, allowing them to readily pass through cell membranes, The hydroxylated and aquated forms are of much lower lipid solubility (particularly at neutral or slightly alkaline pH), which accounts for the general inactivity of these forms of the drug. Further, the elimination of cisplatin takes place much more rapidly than carboplatin, which accounts for the different manifestations of the toxicity.

Other toxic effects associated with platinum complexes are neurotoxicity, ototoxicity (particularly with cispiatin), GI distress, mainly nausea and vomiting, and others.

### J. Other Drugs

Similar toxic effects, namely the hematologic toxicities due to bone marrow suppression, GI distress, hypersensitivity, renal toxicity, liver toxicity, mucositis and others, are associated to some degree with most antineoplastic agents. Since the objectives of any cancer therapy program necessarily include prolonging the patient's life, as well as improving the quality of life, manifestations of toxicity are always carefully weighed against the alternatives.

### 3. Antineoplastic Agent Derivatives

The antineoplastic agent derivatives of the present invention comprise an antineoplastic agent that has been modified by attaching a reactive group. The reactive group may be attached to the antineoplastic agent via a linking group, or optionally without using a linking group. In general, the antineoplastic agent derivative comprises the antineoplastic agent molecule and a linking group together with a reactive group capable of reaction with a functionality on a blood component. Antineoplastic agent derivatives may be administered *in vivo* such that conjugation with blood components occurs *in vivo,* or they may be first conjugated to blood components *in vitro* and the resulting conjugated antineoplastic agents (as defined below) administered *in vivo.*

To form covalent bonds with functionalities on a protein, one may use as a reactive group a wide variety of active carboxyl groups, particularly esters, where the hydroxyl moiety is physiologically acceptable at the levels required to derivatize the antineoplastic agent. White a number of different hydroxyl groups may be employed in these linking agents, the most convenient would be N-hydroxysuccinimide (NHS), and N-hydroxy-suffosuceinimide (sulfo-NHS).

Primary amines are the principal targets for NHS esters as diagramed in schematic 1A below. Accessible α-amino groups present on the N-termini of proteins react with NHS esters. However, α-amino groups on a protein may not be desirable or available for the NHS coupling. While five amino acids have nitrogen in their side chains, only the ε-amine of lysine reacts significantly with NHS esters. An amide bond is formed when the NHS ester conjugation reaction reacts with primary amines releasing N-hydroxysuccinimide as demonstrated in schematic 1A below.

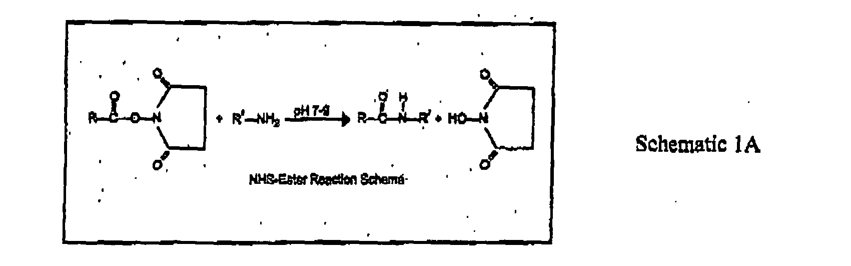

In the preferred embodiments of this invention, the functionality on the protein will be a thiol group and the reactive group will be a maleimido-containing group such as gamma-maleimide-butyralamide (GMBA) or MPA. The maleimido group is most selective for sulfhydryl groups on proteins when the pH of the reaction mixture is kept between 6.5 and 7.4 as shown in schematic 1B below. At pH 7.0, the rate of reaction of maleimido groups with sulfhydryls is 1000-fold faster than with amines. A stable thioether linkage between the maleimido group and the sulfhydryl is formed which cannot be cleaved under physiological conditions.

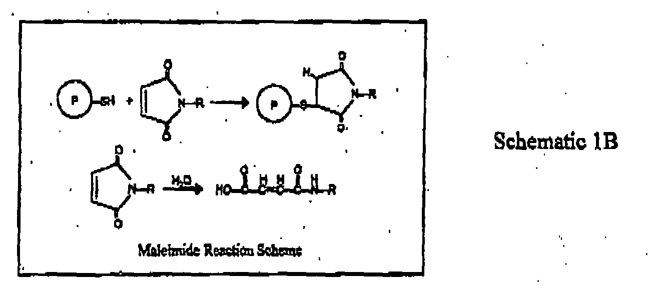

The manner of producing the antineoplastic agent derivatives of the present invention will vary widely, depending upon the nature of the various elements comprising the molecule. The synthetic procedures will be selected so as to be simple, provide for high yields, and allow for a highly purified product. Normally, the chemically reactive group will be created as the last stage, for example, with a carboxyl group, esterification to form an active ester will be the last step of the synthesis. Each antineoplastic agent selected to undergo the derivatization with a linker and a reactive agent, and will be modified according to the following criteria:
First, the location of the pharmacophore region is determined according to Structure-Activity-Relationship (SAR) studies previously published by experts in the field. For example, taxol and docetaxel structure activity relations have been studied and are well explored. Bioconjugates have been attached at the C2' position in the form of an ester and activity was maintained (F. Dosio et al., Journal of Controlled Release, 1997, 47, 293-304; V. Shashoua et al., US 5,795,909 August 18 1998). At the C3' position of docetaxel, the use of a lipophilic amide maintains the activity (F. Gueritte-Voegolein et al., J. Med. Chem., 1991, 34, 992-998; F. Guoritte-Voegalein et al., Europeen Pat. Appl., 253739. July 16 1987 and C. S. Swindell et al., Bloorg. Med. Chem. Lett., 1994, 4, 1531-1536). In the case of doxorubicin, all of the modifications are at the C14 position since this position is the most removed from the pharmacophore region (B. Patelli et al., DE 2627146 December 30 1976; F. Arcamone et al., DE 2557537 July 8 1976; D. H. King et al., Eur. Pat Appl, 294294 A2 December 7 1988). In the case of mitomycin C, the beat position for modification is the N-1a position (N. Umemoto et al., J. Appl. Biochem., 1984, 6,297-307; T. Tanaka et al., Biol. Pharm. Bull., 1996, 19, 774-777 as well as R. L. Barton, Eur. Pat. Appl. 368668 November 10 1988). In the case of vincristine, the best position for modification is the C23 position (as R. L. Barton, Eur. Pat. Appl. 368668 November 10 1988).
Then, the antineoplastic agent is modified at a site sufficiently far away from the pharmacophor region to prevent a potential binding interference between the modified drug and the biological receptor and such that the antineoplastic agent substantially retains its therapeutic activity, (i.e. the therapeutic activity is reduced by no more than 10 fold).
Finaly, keeping constant the site of chemical modification, the biological activity of the antineoplastic agent is optimized by modifying the length and nature of the linker. Some examples of specific antineoplastic agent derivatives are given below.

### A. Doxorubicin Derivatives

One example of an antineoplastic agent derivative is a doxorubicin derivative of Formula I: 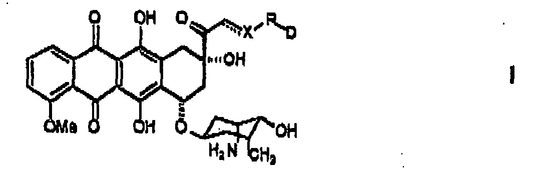 wherein:
X is a sensitive functional group, including but not limited to an ether, thioether, secondary or tertiary amine, secondary or tertiary amide, ester, thioester, imine, hydrazone, semicarbazone, acetal, hemi-acetal, ketal, homi-ketal, aminal, hemi-aminal, sulfonate, sulphate, sulfonamide, sulfonamidate, phosphate, phophoramide, phosphonate, or phosphonamidate, preferably a primary amide;
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₆; and
D is a reactive group as defined above, preferably a maleimide group. The dashed C-X bond signifies the presence, alternatively, of a single or a double bond.

Another example of an antineoplastic agent derivative is a doxorubicin derivative of Formula II: 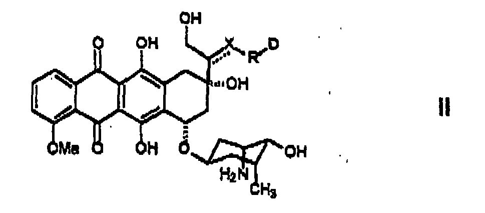 wherein:
X is a sensitive functional group, including but not limited to an ether, thioether, secondary or tertiary amine, secondary or tertiary amide, ester, thioester, imine, hydrazone, semicarbazone, acetal, hemi-acetal, ketal, hemi-ketal, aminal, hemi-aminal, sulfonate, sulphate, sulfonamide, sulforiamidate, phosphate, phophoramide, phosphonate, or phosphonamidate, preferably a primary amide;
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₅; and
D is a reactive group as defined above, preferably a maleimide group. The dashed C-X bond signifies the presence, alternatively, of a single or a double bond.

Another example of an antineoplastic agent derivative is a doxorubicin derivative of Formula III; 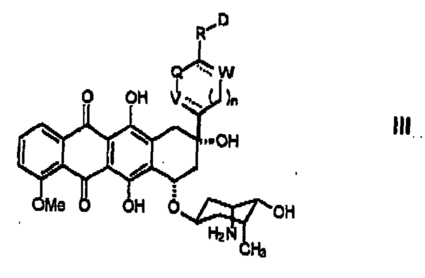 wherein:
V is O, N, S, or C;
W is O, N, S, or C;
n is 0 or 1; and
Q is a nullity, or an oxygen, sulfur, secondary or tertiary amine, secondary or tertiary amide, oxygenated phophorus, or a substituted or unsubstituted carbon atom or nothing (preferably, V is an oxygen, Q is a nullity and W is a primary amine);
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably -CH₂-; and
D is a reactive group as defined above, preferably a maleimide group. The dashed C-Q and C-V bonds signifies the presence, alternatively, of a single or a double bond.

### B. Taxol Derivatives

For the purposes of this invention, derivatized taxol was synthesized by attaching the reactive group at the open position, *i.e.* the free alcohols, even though there is evidence of reduced activity. Bioconjugates have been attached at the C2' position in the form of an ester and activity was maintained (F. Dosio et al., Journal of Controlled Release, 1997, 47,293-304). We believe that this bond is too lablle and can be cleaved in vivo (Magri et al. J. Med. Chem., 1989,32,788-792).

One example of a taxol derivative according to the present invention is a compound of Formula IV; 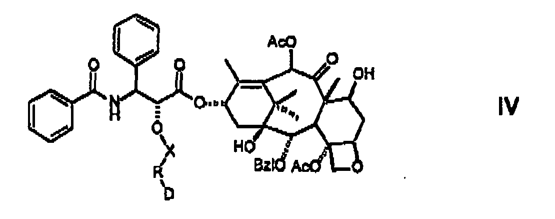 wherein:
X is a sensitive functional group, Including but not limited to an ether, thioether, secondary or tertiary amine, secondary or tertiary amide, ester, thioester, Imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, or phosphonamidate, preferably -NH- or -CO-;
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₆ or -(O-CH₂-CH₂-O)₁₋₂-CH₂CH₂-; and
D is a reactive group as defined above, preferably a maleimide group.

Another example of a taxol derivative according to the present invention is a compound of Formula V: 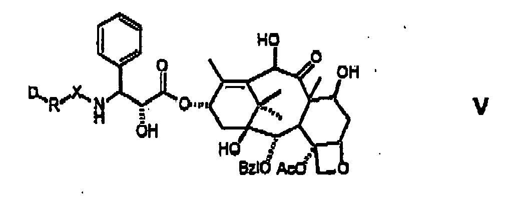 wherein:
X is a sensitive functional group, including but not limited to an ether, thioether, secondary or tertiary amine, secondary or tertiary amide, ester, thioester, imine, hydrazone, semicarbazone, acetal, heml-acetal, ketal, heml-ketal, aminal, hemi-aminal, sulfonate, sulphate, sulfonamide, sulfonamidate, phosphate, phophoramide, phosphonate, or phosphonamidate, preferably a -CO- (amide);
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₆; and
D is a reactive group as defined above, preferably a maleimide group.

### C. Methotrexate Derivatives

One example of a methotrexate derivative according to the present invention is a compound of Formula VI: 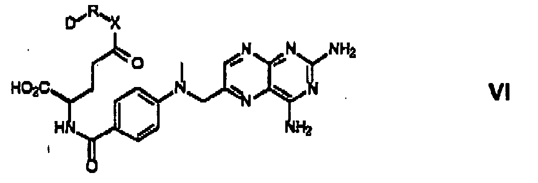 wherein:
X is a sensitive functional group, including but not limited to an ether, thioether, secondary or tertiary amine, secondary or tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, or phosphonamidate, preferably -NH- (primary amide);
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₆; and
D is a reactive group as defined above, preferably a maleimide group.

Another example of a methotrexate derivative according to the present invention is a compound of Formula VII: 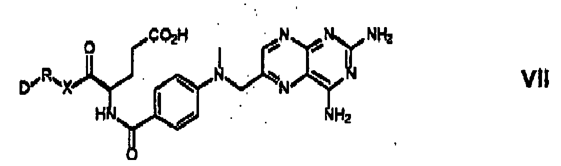 wherein:
X is a sensitive functional group including but not limited to a secondary or tertiary amine, secondary or tertiary amide, ester, thioester, imine, hydrazone, semicarbazone, acetal, hemi-acetal, ketal, hemi-ketal, aminal, hemi-amfnal, sulfonate, sulphate; sulfonamide, sulfonamidate, phosphate, phophoramide, phosphonate, or phosphonamidate, preferably -NH-;
R is an optional linking group as defined above, for example R can be (but is not limited to) a nullity (in which, case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₆: and
D is a reactive group as defined above, preferably a maleimide group.

### D. Hydroxyurea Derivatives

One example of a hydroxyurea derivative according to the present invention is a compound of Formula VIII; 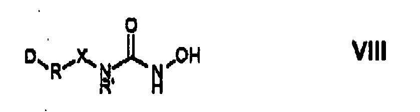 wherein:
X is a sensitive functional group, including but not limited to an ether, thioether, secondary or tertiary amine, secondary or tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, or phosphonamidate, preferably -CO- (primary amide);
R' is an alkyl C₁₋₆, fluoro alkyl C₁₋₆, substituted or unsubstituted aryl or heteroaryl;
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₆; and
D is a reactive group as defined above, preferably a maleimide group.

### E. Tamoxifen Derivatives

One example of a tamoxifen derivative according to the present invention is a compound of Formula IX: 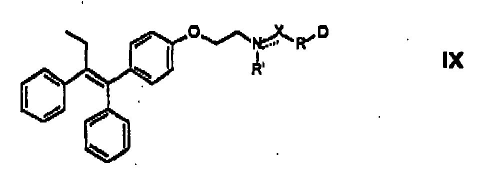 wherein:
X is a sensitive functional group, including but not limited to an ether, thioether, secondary or tertiary amine, secondary or tertiary amide, ester, thioester, imine, hydrazone, semicarbazone, acetal, hemi-acetal, ketal, hemi-ketal, aminal, hemi-eminal, sulfonate, sulphate, sulfonamide, sulfonamidate, phosphate, phophoramide, phosphonate, or phosphonamidate, preferably -NH-CO- (primary amide);
R' is a hydrogen, alkyl, aryl, or acyl, preferably a methyl;
R is an optional linking group as defined above, for example R can be (but is not limited to) a nullity (In which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₈; and
D is a reactive group as defined above, preferably a maleimide group.

### F. Mitomycin C Derivatives

One example of a mitomycin C derivative according to the present invention is a compound of Formula X: 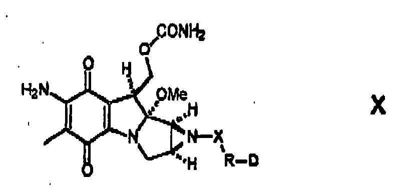 wherein:
X is a sensitive functional group, including but not limited to an ether, thioether, secondary or tertiary amine, secondary or tertiary amide, ester, thioester, imine, hydrazone, semicarbazone, acetal, hemi-nestal, ketal, hemi-ketal, aminal, hemi-aminal, sulfonate, sulphate, sulfonamide, sulfonamidate, phosphate, phophoramide, phosphonate, or phosphonamidate, preferably -CO- (amide);
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either 0 or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₆; and
D is a reactive group as defined above, preferably a maleimide group.

### G. 5-Fluorouracil

The compound in this invention which is the product of the derivation of 5-fluorouracil is a mixture of four regioisomers. These four regloisomers are compounds of Formulae XI A, XI B, XI C and XI D. 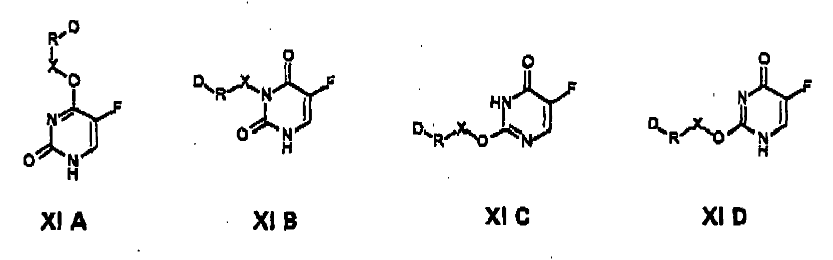 wherein:
X is a sensitive functional group, including but not limited to an ether, thioether, secondary or tertiary amine, secondary or tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, or phosphonamidate, preferably -CO- (ester or amide);
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₆H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole, preferably an alkyl chain C₁₋₅; and
D is a reactive group as defined above, preferably a maleimide group.

### H. Vincristine

One example of a vincristine derivative according to the present invention is a compound of Formula XII: 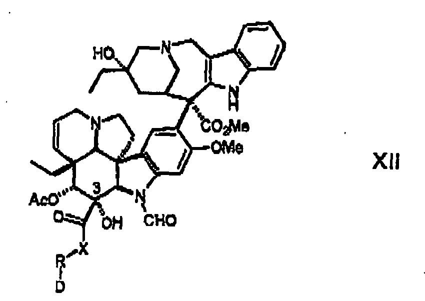 wherein:
X is a sensitive functional group including but not limited to a secondary or tertiary amine, secondary or tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, or phosphonamidate;
R is an optional linking group as defined above, for example R can be (but is not limited to) a nullity (in which case X is directly bonded to D), any alkyl chain C₁₋₁₀, any fluoroalkyl C₁₋₁₀ or any combination of fluorosubstituted alkyl chain C₁₋₁₀, any ether or thioether containing alkyl or fluoroalkyl chains such as -(Z-CH₂CH₂-Z)ₙ-, -(Z-CF₂CH₂-Z)ₙ-, -(Z-CH₂CF₂-Z)ₙ-, where n=1-4 and Z is either O or S, ortho, meta or para disubstituted benzene with structure like -Y-C₈H₄-, -Y-C₆H₄-Y-, where Y is any combination of CH₂, O, S, NH, NR [R=H, alkyl, acyl], disubstituted heterocycles such as furan, thiophene, pyran, oxazole, or thiazole; and
D is a reactive group as defined above, preferably a maleimide group.

### 4. Conjugated Antineoplastic Agents

After an antineoplastic agent Is derivatized according to the present invention, it is used to form conjugated antineoplastic agents either *in vivo* or *in vitro.* Such a conjugate comprises an antineoplastic agent derivative conjugated to a blood component via a covalent bond formed between the reactive group of the antineoplastic agent derivative (attached to the antineoplastic agent derivative either with or without a linking group), and the functionality on the blood component. The antineoplastic agent derivatives of the invention may be used for conjugation by specific labeling or non-specific labeling of blood components, as described in more detail below.

### A. Specific Labeling

Preferably, the antineoplastic agent derivatives of this invention are designed to specifically react with thiol groups on mobile blood proteins. Such reaction is preferably established by the covalent bonding of an antineoplastic agent derivatized with a maleimide (e.g. prepared from GMBS, MPA or other maleimides) to a thiol group on a mobile blood protein such as serum albumin or IgG.

Under certain circumstances, specific labeling with maleimides offers several advantages over non-specific labeling of mobile proteins with groups such as NHS and sulfo-NHS. Thiol groups are less abundant *in vivo* than amino groups. Therefore, the maleimide derivatives of this invention will covalently bond to fewer proteins. For example, in albumin (the most abundant blood protein) there is only a single thiol group. Thus, antineoplastic agent-maleimide-albumin conjugates will tend to comprise approximately a 1:1 molar ratio of antineoplastic agent to albumin. In addition to albumin, IgG molecules (class II) also have free thiols. Since IgG molecules and serum albumin make up the majority of the soluble protein in blood they also make up the majority of the free thiol groups in blood that are available to covalently bond to maleimide-antineoplastic agents.

Further, even among free thiol-containing blood proteins, specific labeling with maleimides leads to the preferential formation of antineoplastic agent-maleimide-albumin conjugates, due to the unique characteristics of albumin itself. The single free thiol group of albumin, highly conserved among species, is located at amino acid residue 34 (Cys³⁴). It has been demonstrated recently that the Cys³⁴ of albumin has increased reactivity relative to free thiols on other free thiol-containing proteins. This is due in part to the very low pK value of 5.5 for the Cys³⁴ of albumin. This is much lower than typical pK values for cysteines residues in general, which are typically about 8. Due to this low pK, under normal physiological conditions Cys³⁴ of albumin is predominantly in the ionized form, which dramatically increases its reactivity. In addition to the low pK value of Cys³⁴, another factor which enhances the reactivity of Cys³⁴ is its location, which is in a crevice close to the surface of one loop of region V of albumin. This location makes Cys³⁴ very available to ligands of all kinds, and is an important factor in Cys³⁴'s biological role as free radical trap and free thiol scavenger. These properties make Cys³⁴ highly reactive with antineoplastic agent-maleimides, and the reaction rate acceleration can be as much as 1000-fold relative to rates of reaction of antineoplastic agent-maleimides with other free-thiol containing proteins.

Another advantage of antineoplastic agent-maleimide-albumin conjugates is the reproducibility associated with the 1:1 loading of antineoplastic agent to albumin specifically at Cys³⁴. Other techniques, such as glutaraldehyde, DCC, EDC and other chemical activations of, for example, free amines lack this selectivity. For example, albumin contains 52 lysine residues, 25-30 of which are located on the surface of albumin and accessible for conjugation. Activating these lysine residues, or alternatively modifying antineoplastic agents to couple through these lysine residues, results in a heterogenous population of conjugates. Even if 1:1 molar ratios of antineoplastic agent to albumin are employed, the yield will consist of multiple conjugation products, some containing 0,1,2 or more antineoplastic agents per albumin, and each having antineoplastic agents randomly coupled at any one of the 25-30 available lysine sites. Given the numerous combinations possible, characterization of the exact composition and nature of each batch becomes difficult, and batch-to-batch reproducibility is all but impossible, making such conjugates less desirable as a therapeutic. Additionally, while it would seem that conjugation through lysine residues of albumin would at least have the advantage of delivering more therapeutic agent per albumin molecule, studies have shown that a 1:1 ratio of therapeutic agent to albumin is preferred. In an article by Stehle, et al., "The Loading Rate Determines Tumor Targeting Properties of Methotrexate-Albumin Conjugates in Rats," Anti-Cancer Drugs, Vol. 8, pp. 677-685 (1997), incorporated herein in its entirety, the authors report that a 1:1 ratio of the anti-cancer methotrexate to albumin conjugated via glutaraldehyde gave the most promising results. These conjugates were taken up by tumor cells, whereas conjugates bearing 5:1 to 20:1 methotrexate molecules had altered HPLC profiles and were quickly taken up by the liver *in vivo.* It is postulated that at these higher ratios, conformational changes to albumin diminish its effectiveness as a therapeutic carrier.

Through controlled administration of malelmide-antineoplastic agents *in vivo,* one can control the specific labeling of albumin and IgG *in vivo.* In typical administrations, 80-90% of the administered maleimide-antineoplastic agent will label albumin and less than 5% will label IgG. Trace labeling of free thiols such as glutathione will also occur. Such specific labeling is preferred for *in vivo* use as it permits an accurate calculation of the estimated half-life of the administered agent.

In addition to providing controlled specific *in vivo* labeling, maleimlde-antineoplastic agent can provide specific labeling of serum albumin and IgG *ex vivo.* Such *ex vivo* labeling Involves the addition of maleimide-antineoplastic agents to blood, serum or saline solution containing serum albumin and/or IgG. Once modified *ex vivo* with maleimide-antineoplastic agents, the blood, serum or saline solution can be readministered to the blood for *in vivo* treatment.

In comparison to NHS-antineoplastic agents, maleimide-antineoplastic agents are generally stable in the presence of aqueous solutions and in the presence of free amines. Since maleimide-antineoplastic agents will only react with free thiols, protective groups are generally not necessary to prevent the maleimide-antineoplastic agents from reacting with itself. In addition, the increased stability of the antineoplastic agent permits the use of further purification steps such as HPLC to prepare highly purified products suitable for *in vivo* use. Lastly, the increased chemical stability provides a product with a longer shelf life.

### B. Non-Specific Labeling

The antineoplastic agents of the invention may also be derivatized for non-specific labeling of blood components. Bonds to amino groups will generally be employed, particularly with the formation of amide bonds for non-specific labeling. To form such bonds, one may use as a chemically reactive group coupled to the antineoplastic agent a wide variety of active carboxyl groups, particularly esters, where the hydroxyl moiety is physiologically acceptable at the levels required. While a number of different hydroxyl groups may be employed in these linking agents, the most convenient would be N-hydroxysuccinimide (NHS) and N-hydroxy-sulfosuccinimide (sulfo-NHS).

Other linking agents which may be utilized are described in U.S. Patent 5,612,034, which is hereby incorporated herein.

The various sites with which the chemically reactive groups of the non-specific antineoplastic agents may react *in vivo* include cells, particularly red blood cells (erythrocytes) and platelets, and proteins, such as immunoglobulins, including IgG and IgM, serum albumin, ferritin, steroid binding proteins, transferrin, thyroxin binding protein, α-2-macroglobulin, and the like. Those receptors with which the antineoplastic agent derivatives react, which are not long-lived, will generally be eliminated from the human host within about three days. The proteins indicated above (including the proteins of the cells) will remain in the bloodstream at least three days, and may remain five days or more (usually not exceeding 60 days, more usually not exceeding 30 days) particularly as to the half life, based on the concentration in the blood.

For the most part, reaction will be with mobile components in the blood, particularly blood proteins and cells, more particularly blood proteins and erythrocytes. By "mobile" is intended that the component does not have a fixed situs for any extended period of time, generally not exceeding 5 minutes, more usually one minute, although some of the blood components may be relatively stationary for extended periods of time. Initially, there will be a relatively heterogeneous population of labeled proteins and cells. However, for the most part, the population within a few days after administration will vary substantially from the initial population, depending upon the half-life of the labeled proteins in the blood stream. Therefore, usually within about three days or more, IgG will become the predominant labeled protein in the blood stream.

Usually, by day 5 post-administration, IgG, serum albumin and erythrocytes will be at least about 60 mole %, usually at least about 75 mole %, of the conjugated components in blood, with IgG, IgM (to a substantially lesser extent) and serum albumin being at least about 50 mole %, usually at least about 75 mole %, more usually at least about 80 mole %, of the non-cellular conjugated components.

The desired conjugates of non-specific antineoplastic agents to blood components may be prepared *in vivo* by administration of the antineoplastic agents directly to the patient, which may be a human or other mammal. The administration may be done in the form of a bolus or introduced slowly over time by infusion using metered flow or the like.

If desired, the subject conjugates may also be prepared *ex vivo* by combining blood with antineoplastic agent derivatives of the present invention, allowing covalent bonding of the antineoplastic agent derivatives to reactive functionalities on blood components and then returning or administering the conjugated blood to the host. Moreover, the above may also be accomplished by first purifying an individual blood component or limited number of components, such as red blood cells, immunoglobulins, serum albumin, or the like, and combining the component or components *ex vivo* with the chemically reactive antineoplastic agents. The labeled blood or blood component may then be returned to the host to provide *In vivo* the subject therapeutically effective conjugates. The blood also may be treated to prevent coagulation during handling *ex vivo.*

### 5. Therapeutic Uses of Antineoplastic Agent Derivatives

The compounds of the invention, including but not limited to those specified in the examples, possess anti-cancer activity. As derivatives of antineoplastic agents having anti-cancer activity, the compounds of the present invention are useful in the treatment of a variety of diseases, for example primary and metastatic solid tumors and carcinomas of the breast; colon; rectum; lung; oropharynx; hypopharynx; esophagus; stomach. Pancreas; liver; gallbladder; bile ducts; small intestine; urinary tract including kidney, bladder and urothelium; female genital tract including cervix, uterus, endometrium, ovaries, choriocarcinoma and festational trophoblastic disease; male genital tract including prostate, seminal vesicles, testes and germ cells tumors; endocrine glands including thyroid, adrenal and pituitary; skin including hmangiomas, melanomas, sarcomas arising from bone of soft tissues and Karposi's sarcoma, Wilm's tumor, rhabdomyosarcoma; tumor of the head and neck, brain, nerves, eyes, and meninges including astrocytomas, gliomas, giloblastomas, retinobiastomas, neuromas, neuroblastomas; tumors of the bone marrow and hematopoeitic tumors, solid tumors arising from hematopoietic malignancies such as leukemias and Including chloromas, plasmocytomas, plagues and tumors of mycosis fungoides and cutaneous T-cell lymphoma/leukemia; acute lymphotic, actute granulocytic and chronic granulocytic leukemia; lymphomas including both Hodgkin's and non-Hodgkin's lymphomas: prophylaxis of autoimmune diseases including rheumatoid, immune and degenerative arthritis; ocular diseases including diabethic retinopathy; retinopathy of prematurity; corneal graft rejection, retrolental fibroplasia, neovascular glaucoma, rubeosis, retinal neovascularization due to macular degeneration and hypoxia; abnormal neovascularization conditions of the eye; skin diseases including psoriasis; blood vessel diseases including hemagiomas and capillary proliferatrion within atherosclerotic plaques; myocardial angiogenesis; plaque neovascularization; hemophiliac joints; angiofibroma; wound granulation; dieases charadterized by excessive or abnormal stimulation of endothelial cells including intestinal adhesion, Crohn's disease, atheroscelrosis, scleroderma and hypertrophic scars and diseases which have angiogenesis as a pathological consequence including ulcers (Helicobacter pilori); rheumatoid arthritis, osteogenic sarcoma, osteoarthritis, osteopenias such as osteoporosis, periodontitis, gingivitis, corneal epidermal or gastric ulceration, and tumor metastasis, invasion and growth, retinopathies, wound healing (ocular inflammation, soft and osseous tissue disease, gingivitis/periodontal disease), vascular disease (restenosis) annuerysm inflammation, autoimmune diseases, and rare cancers such as choriocarcinoma.

The compounds of the present invention may also be useful for the prevention of metastases from the tumors described above either when used alone or in combination with radiotherapy and /or other chemotherapeutic treatments conventionally administered to patients for treating angiogenic diseases. For example, when used in the treatment of solid tumors, compounds of the present invention may be administered with chemotherapeutic agents such as alpha-inferon, COMP (cyclophosphamnide, vincristine, methotraxate and prednisone), etoposide, mBACOD (methotraxate, bleomycin, doxorubicin, cyclophosphamide, vincristine and dexamethasone), PROMACE/MOPP (prednisone, methotrexate, doxirubicin, cyclophaophamide, taxol, etoposide/mechloetamine, vincristine, prednisone and procarbazine), vincristine, vinblastine, angioinhibins, TNP-470, pentosan polysulfate, platelet factar-4, angiostatin, LM-609, SU-1 01, CM-101, techgalan, thalidomide, SP-PG and the like. Other chemotherapeutic agents include alkylating agents such as nitrogen mustards Including mechloethamine, melphanchloambucil, cyclophaosphamide, and ifosfamide; nirrosdoureas including carmustine, lomustine, semustine and streptozocin; alkyl sulfonates icluding busulfan; triazines including dacarbazine; ethyenimines including thiotepa na dhexamethylmelanine; folic acid analogs including methotraxate; pyrimidine analogs including 5-FU, cytosine arabinoside; purine analogs including 6-mercaptopurine and 6-thloguanins; antitumor antibiotics including actinomycin D; the anthraqcyclines including doxorubicin, bleomycin, mitomycin C and methramycin; hormones and hormones antagonists including tamoxifen and corticosteroids and mioscellaneous agnets including cisplatin and brequinar; fragments of plasminogen (kringle-5) as well as fragments from other integrin-binding substrates. For instnace, a tumor may be treated conventionally with surgery, radiation or chemiotherapy and administration of antineoplastic agent derivatives to extend the dormancy of micrometastasis and to inhibit the growth of any residual primary tumor.

### 6. Administration of Antineoplastic Agent Derivatives

in accordance with the present invention, antineoplastic agent derivatives may be prepared for administration by any route or administered by any route. The antineoplastic agent derivatives will be administered in a physiologically acceptable medium, e.g. deionized water, phosphate buffered saline (PBS), saline, aqueous ethanol or other alcohol, plasma, proteinaceous solutions, mannitol, aqueous glucose, alcohol, vegetable oil, or the like. Other additives which may be included Include buffers, where the media are generally buffered at a pH in the range of about 5 to 10, where the buffer will generally range in concentration from about 50 to 250 mM, salt, where the concentration of salt will generally range from about 5 to 500 mM, physiologically acceptable stabilizers, and the like. The compositions may be lyophilized for convenient storage and transport.

The subject antineoplastic agent derivatives will for the most part be administered parenterally, such as intravascularly (IV), intraarterially (IA), intramuscularly (IM), subcutaneously (SC), or the like. Administration may in appropriate situations be by transfusion. In some instances, where reaction of the functional group is relatively slow, administration may be oral, nasal, vaginal, rectal, transdermal or aerosol, where the nature of the conjugate allows for transfer to the vascular system. Usually a single injection will be employed although more than one injection may be used, if desired. The antineoplastic agent derivatives may be administered by any convenient means, including syringe, trocar, catheter, or the like. The particular manner of administration will vary depending upon the amount to be administered, whether a single bolus or continuous administration, or the like. Preferably, the administration will be intravascularly, where the site of introduction is not critical to this invention, preferably at a site where there is rapid blood flow, e.g., intravenously, peripheral or central vein. Other routes may find use where the administration is coupled with slow release techniques or a protective matrix. The intent is that the antineoplastic agent, analog or derivative be effectively distributed in the blood, so as to be able to react with the blood components. The concentration of the conjugate will vary widely, generally ranging from about 1 pg/ml to 50 mg/ml. The total administered intravascularly will generally be in the range of about 0.1 mg/ml to about 10 mg/ml, more usually about 1 mg/ml to about 5 mg/ml.

By bonding to long-lived components of the blood, such as immunoglobulin, serum albumin, red blood cells and platelets, a number of advantages ensue. The activity of the antineoplastic agent compound is extended for days to weeks. Only one administration need be given during this period of time. Greater specificity can be achieved, since the active compound will be primarily bound to large molecules, where it is less likely to be taken up intracellularly to interfere with other physiological processes.

The blood of the mammalian host may be monitored for the presence of the antineoplastic agent compound one or more times. By taking a portion or sample of the blood of the host, one may determine whether the antineoplastic agent has become bound to the long-lived blood components in sufficient amount to be therapeutically active and, thereafter, the level of antineoplastic agent compound in the blood. If desired, one may also determine to which of the blood components the antineoplastic agent derivative molecule is bound. This is particularly important when using non-specific antineoplastic agent derivatives. For specific maleimide-antineoplastic agent derivatives, it is much simpler to calculate the half life of serum albumin and IgG.

Thus, this invention relates to such conjugates of antineoplastic agent derivatives with blood components, particularly blood proteins such as albumin, as well as methods of administrating them to human and other mammal patients.

### 7. Concentration and Pharmacokinetic Determination of Antineoplatic Agent Derivatives

Another aspect of this invention relates to methods for determining the concentration of the antineoplastic agent derivatives and conjugates in biological samples (such as blood) using antibodies specific to the antineoplastic agent derivatives and conjugates, and to the use of such antibodies as a treatment for toxicity potentially associated with such antineoplastic agents, analogs, and/or their derivatives or conjugates. This is advantageous because the increased stability and life of the antineoplastic agent derivatives *in vivo* in the patient might lead to novel problems during treatment, including increased possibility for toxicity. The use of anti-therapeutic agent antibodies, either monoclonal or polyclonal, having specificity for a particular antineoplastic agent derivative can assist in mediating any such problem. The antibody may be generated or derived from a host immunized with the particular antineoplastic agent derivative, or with an immunogenic fragment of the agent, or a synthesized immunogen corresponding to an antigenic determinant of the agent. Preferred antibodies will have high specificity and affinity for native, derivatized and conjugated forms of the antineoplastic agent derivative. Such antibodies can also be labeled with enzymes, fluorochromes, or radiolabels.

Antibodies specific for derivatized antineoplastic agents may be produced by using purified antineoplastic agent for the induction of derivatized antineoplastic agent-specific antibodies. By induction of antibodies, it is intended not only the stimulation of an immune response by injection into animals, but analogous steps in the production of synthetic antibodies or other specific binding molecules such as screening of recombinant immunoglobulin libraries. Both monoclonal and polyclonal antibodies can be produced by procedures well known in the art.

The anti-therapeutic agent antibodies may be used to treat toxicity induced by administration of the antineoplastic agent derivative and may be used *ex vivo* or *in vivo. Ex vivo* methods would include immuno-dialysis treatment for toxicity employing anti-therapeutic agent antibodies fixed to solid supports. *In vivo* methods include administration of anti-therapeutic agent antibodies in amounts effective to induce clearance of sntibody-agent complexes.

The antibodies may be used to remove the antineoplastic agent derivatives from a patient's blood *ex vivo* by contacting the blood with the antibodies under sterile conditions. For example, the antibodies can be fixed or otherwise immobilized on a column matrix and the patents blood can be removed from the patient and passed over the matrix. The antineoplastic agent derivatives or conjugates, will bind to the antibodies and the blood containing a low concentration of the antineoplastic agent derivative or conjugate, then may be returned to the patient's circulatory system. The amount of antineoplastic agent compound removed can be controlled by adjusting the pressure and flow rate. Preferential removal of the antineoplastic agent derivatives and conjugates from the plasma component of a patient's blood can be effected, for example, by the use of a semipermeable membrane, or by otherwise first separating the plasma component from the cellular component by ways known in the art prior to passing the plasma component over a matrix containing the anti-therapeutic antibodies. Alternatively the preferential removal of antineoplastic agent-conjugated blood cells, including red blood cells, can be effected by collecting and concentrating the blood cells in the patient's blood and contacting those cells with fixed anti-therapeutic antibodies to the exclusion of the serum component of the patients blood.

The anti-therapeutic antibodies can be administered *in vivo,* parenterally, to a patient that has received the antineoplastic agent derivatives or conjugates for treatment. The antibodies will bind the antineoplastic agent compounds and conjugates. Once bound the antineoplastic agent derivative activity will be hindered if not completely blocked thereby reducing the biologically effective concentration of antineoplastic agent derivative in the patient's bloodstream and minimizing harmful side effects. In addition, the bound antibody-antineoplastic agent complex will facilitate clearance of the antineoplastic agent compounds and conjugates from the patient's blood stream.

To determine the pharmacokinetic profile of the antineoplastic agent derivatives of this invention, ¹⁴C radio labeling is the most efficient method since such small quantities of drug are necessary to show efficacy after administration. The pharmacokinetic profile of the compounds described in this invention is interpreted in terms of the area under the curve (AUC). The AUC is measured using a ¹⁴C labeled drug available commercially in the cases where the reactive group is attached in one step or when it is attached with a hydrolytically unstable bond. The cases involving mufti-stop syntheses have the ¹⁴C radio label on the reactive group and attached to the antineoplastic agent either in the last step or very late in the synthesis. Pharmacokinetic evaluation is done by first injecting a physiologically suitable solution at a suitable concentration of the radio labeled antineoplastic agent derivative into rats via intra peritoneal In the thoracic area or preferably via intra vinous in the tail vein or the neck artery in a single bolus dose. Measurements are taken at the following time points: -2, 0, 0.25, 0.5, 1, 2, 4,24, 48 h where a suitable number of specimens are used at each time point. The AUC is evaluated from the radio active counts plotted against time for blood, and various organs which may or may not include the liver, kidneys, heart, lungs, brain, adipose, muscles and bone. Even if *in vitro* activity is reduced, the improved pharmacokinetic profile improves the pharmacodynamic effect and results in greater efficacy and reduced side effects.

The invention having been fully described is now exemplified by the following non-limiting examples. The present invention will be more readily understood by referring to the following examples which are given to illustrate the invention rather than to limit its scope.

### Example 1

### Synthesis of an NHS Derivative from a Carboxylic Acid in Absence of Other Sensitive Functionalities in the Molecule

To a solution of compound drug to be modified (1 mmol) containing a carboxylic acid in absence of other sensitive functionalities in the molecule and N-hydroxysuccinimide (1.1 mmol) In anhydrous CH₂Cl₂ (10 mL) is added EDC (2.2 mmol). The solution is stirred at room temperature for 20 hours or until complete. The reaction is then washed with water, saturated sodium chloride, dried with anhydrous sodium sulfate, filtered and concentrated *in vacuo.* The residue is dissolved in minimum amount of solvent and purified by chromatography or recrystallized from the appropriate solvent system to give the NHS derivative.

### Example 2

### Synthesis of an NHS Derivative from a Molecule Containing an Amino and/or a Thiol Functionality and a Carboxylic Acid

When a free amino or thiol group is present in the molecule, it is preferable to protect these functional groups prior to perform the addition of the NHS derivative. For instance, if the molecule contains a free amino group, a transformation of the amine into a Fmoc or preferably into a tBoc protected amine is necessary prior to perform the chemistry described in Example 1. The amine functionality will not be deprotected after preparation of the NHS derivative. Therefore this method applies only to a compound whose amine group is not required to be freed to induce a pharmacological desired effect. If the amino group needs to be freed to retain the original biological properties of the molecule, then another type of chemistry described in Example 3-6 has to be performed.

### Example 3

### Synthesis of an NHS Derivative from a Molecule Containing an Amino or a Thiol Functionality and No Carboxylic Acid

When the selected molecule contains no carboxylic acid, an array of bifunctional linker can be used to convert the molecule into a reactive NHS derivative. For instance, ethylene glycol-bis(succinimydylsuccinate) (EGS) and triethylamine dissolved in DMF and added to the free amino containing molecule (with a ratio of 10:1 in favor of EGS) will produce the mono NHS derivative.

To produce an NHS derivative from a thiol derivatized molecule, one can use N-[4-malsimidobutyryloxy]succinimide ester (GMBS) and triethylamine in DMF. The maleimido group will react with the free thiol and the NHS derivative will be purified from the reaction mixture by chromatography on silica or by HPLC.

### Example 4

### Synthesis of an NHS Derivative from a Molecule Containing Multiple Chemical Functionalities

Each case will have to be analyzed and solved in a different manner. However, thanks to the large array of protecting groups and bifunctional linkers that are commercially available, this invention is applicable to any molecule with preferably one chemical step only to derivatize the molecule (as described in Example 1 or 3) or two steps (as described in Example 2 and involving prior protection of a sensitive group) or three steps (protection, activation and deprotection). Under exceptional circumstances only, would we require to use multiple steps (beyond three steps) synthesis to transform a molecule into an active NHS or maleimide derivative.

### Example 5

### Synthesis of a Maleimide Derivative from a Molecule Containing a Free Amino Group and a Free Carboxylic Acid

To produce an maleimide derivative from a amino derivatized molecule, one can use N-[4-maleimidobutyryloxy]succinimide ester (GMBS) and triethylamine in DMF. The succinimide ester group will react with the free amino and the maleimide derivative will be purified from the reaction mixture by crystallization or by chromatography on silica or by HPLC.

### Example 6

### Synthesis of a Maleimide Derivative from a Molecule Containing Multiple Other Functionalities and No Free Carboxylic Acid

When the selected molecule contains no carboxylic acid, an array of bifunctional crosslinking reagents can be used to convert the molecule into a reactive NHS derivative. For instance maleimidopropionic acid (MPA) can be coupled to the free amine to produce a maleimide derivative through reaction of the free amine with the carboxylic group of MPA using HBTU/HOBt/DIEA activation in DMF.

### Example 7

### Preparation of Doxorubicin Derivative (MPA-AEEA-Doxcrubicin)

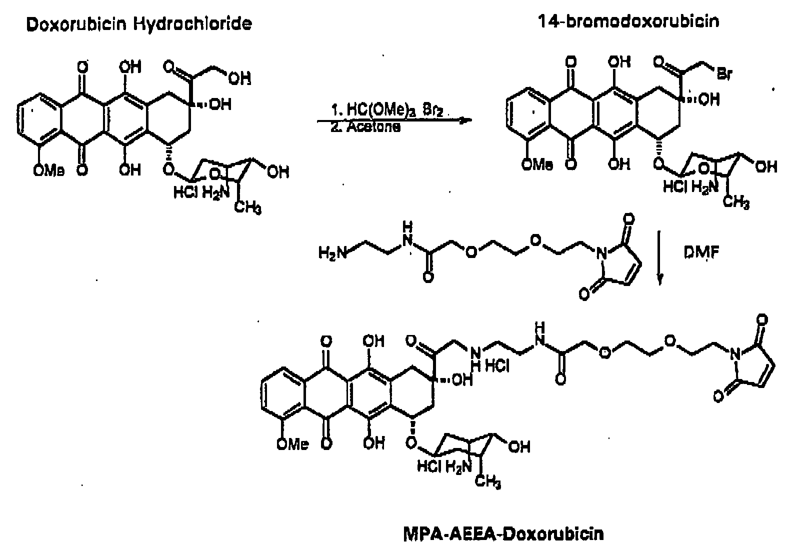

To synthesize the doxorubicin derivative MPA-AEEA-doxorubicin, doxorubicin is initially converted to 14-bromodoxorubicin by using the following procedure:

Doxorubicin hydrochloride (226 mg) is dissolved in 4 mL of anhydrous methanol and is mixed with 0.2 mL of methyl orthoformate and 8 mL of dioxane. After 0.83 mL of bromine solution in methylene chloride (10% w/v) is added, the reaction is carried out at amibient temperature for 40 min. The reaction mixture is poured into 50 mL of dry diethyl ether and the precipitate formed is recovered by centrifugation. The supernatant solution is discarded. After washing twice with 5 mL of dry ether, the precipitate are treated under agitation in 12 mL of acetone at amibient temperature for one hour. The treated deposits are collected by centrifugation, rinsed twice with ether and dried to give 226 mg of red powder of 14-bromodoxorubicine (yield 87%).

The resulting 14-bromodoxorubicin is then converted to the MPA-AEEA-doxorubicin in a single step by treating 14-bromodoxorubicin with amino-AEEA-MPA in *N,N*-dimethylformamide (DMF) in the presence of diisopropylethylamine (DIEA) and heating to 60°C for 1 h. Specifically, 14-Bromodoxorubicin (0.1 mmol) and MEEA-EDA.HCL ([N-(2-amino)ethyl 2-(2-malelmido) ethoxyethoxyacetamide, 0.4 mmol] in DMF (2 mL) and diisopropylethylamine (0.2 mL) are stirred for 2 h. or until complete (by TLC). The reaction mixture is concentrated under reduced pressure and the residue is triturated with ether. The product is purified by preparative HPLC using gradient from 5-60% acetonitrile to give MPA-AEEA-doxorubicin. This procedure is not limited to the use of a nitrogen atom to displace the 14-bromide. One skilled in the art can also use an alcoholate, a secondary amine, a primary or secondary amidate or a primary or secondary sulfonamidate.

### Example 8

### Preparation of Doxorubicin Derivative (MPA-Doxorubicin)

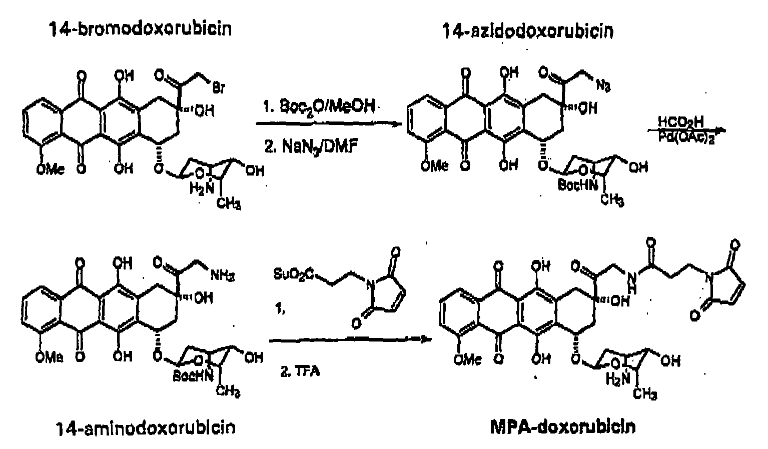

To synthesize MPA-doxorubicin, 14-bromodoxorubicin (0.1 mmol), as synthesized in. Example 7 above, is first converted to the 14-azide by treatment with sodium azide in DMF. To synthesize the azide, the 14-bromodoxodubicin is treated with Boc₂O (0.4 mmol) in MeOH for 60 min. or until complete. The solvent is evaporated and the residue is used for the next step. In the next step, the crude product contained in this residue (0.1 mmol) and NaN₃ (0.4 mmol) in DMF (1 mL) are stirred at room temperature for 16 h. or until complete (TLC). The product is extracted with methylene chloroide and the combined methlene chloride layers are washed with water and dried. The solvent is evaporated and the residue purified by flash column chromatography on silica gel to give the doxorubicin azide.

The 14-azidodoxorubicin is then converted to amino doxorubicin using the following method: 1,3-Propanedithiol (0.3 ml) is added to a solution of doxorubicin azide (0.1 mmol) in DMF (1 ml) at ambient temperature. The reaction, mixture is stirred 16 h or until complete. The solvent is evaporated under vacuum, yielding 14-aminodoxorubicin, which is used for the nest step as a crude product,.

N-methylmorpholine (1 mmol) is added to the methylene chloride solution (10 mL) containing the amino doxorubicin (0.1 mmol) and succinyl 3-malaimidopropanoate (3-MPA). The reaction is stirred for 30 min or until complete. The methylene chloride is washed with water, dired (Na₂SO₄). The solvent is evaporated and the product is purified by flash column chromatography. The product is then treated with TFA (0.5 mL) for 10 min. TFA is evaporated and the residue purified by preparative HPLC to give MPA-doxorubicin.

### Example 9

### Preparation of Doxorubicin Derivative (NHS-C6-doxorubicin)

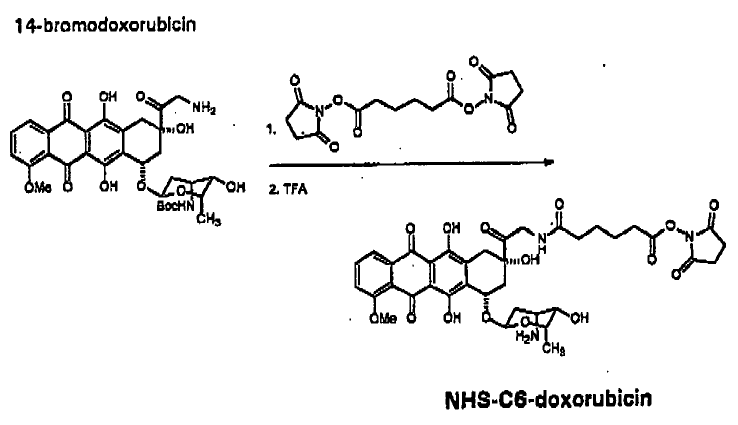

To synthesize NHS-C6-doxorubicin, the 14-aminodoxorubicin as prepared in Example 8 is converted to the six carbon chain by reaction with di-*N*-hydorxysuccinyl adipate (0.14 mmol) in DMF (0.5 ml) at ambient temperature for 1 h. The reaction mixture is then concentrated, the residue is treated with TFA diluted in dichloromethane, again concentrated and the resulting NHS-C6-doxorubicin is purified by HPLC.

### Example 10

### Preparation of Taxol Derivative (MPA-AEE-Taxol)

In order to prevent any cleavage an ether linkage was chosen as a hydrolytically stable bond and the synthesis of MPA-AEE-Taxol is described as follows from taxol.

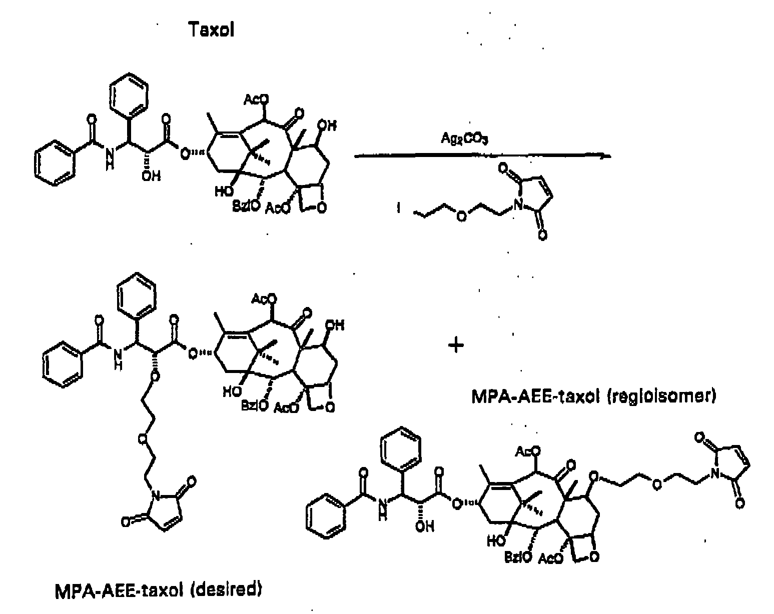

Taxol is converted to the MPA-AEE-taxol derivatives by the following method: Taxol (0.02 mmol) and Ag₂CO₃ (0.1 mmol) in acetonitrile (2 mL) are cooled to 0°C. To the mixture, 2-maleimidoethyl-2'-iodoethylether (MPA-AEE-I)(0.02 mmol) is added. The reaction is stirred for 2 h or until complete. The precipitate is removed by filtration and washed with acetonitrile. The filtrate is evaporated to give a residue, which is purified by preparative HPLC using gradient from 5-60% acetonitrile to give the desired MPA-AEE-taxol derivative as well as the regioisomer.

### Example 11

### Preparation of Taxol Derivative (MPA-Taxol)

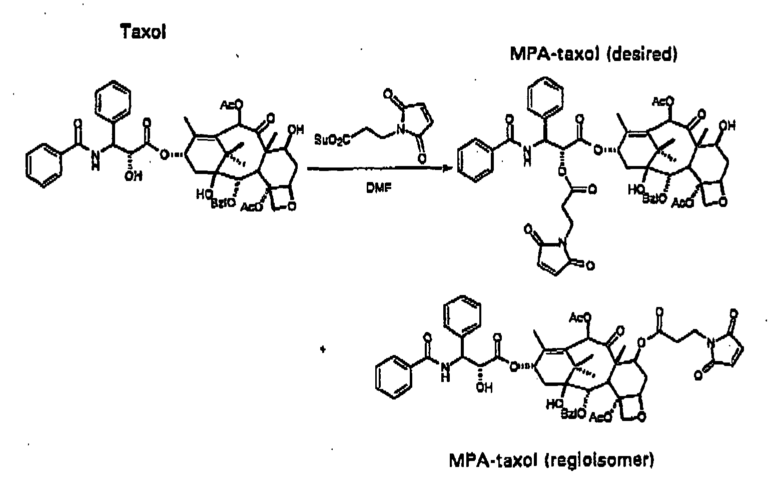

To prepare MPA-taxol, to a solution of Taxol (0.02 mmol) in N,N-dimethylformamide. (1 ml) In the presence of N-methylmorpholine (0.03 mmol), a solution of succinyl 3-maleimidopropanoate (0.025 mmol) is added and the reaction mixture is allowed to stir at ambient temperature for 16 h. The solvent is stripped under vacuum and the residue purified by HPLC using gradient from 5-60% acetonitrile to give the desired MPA-taxol derivative as well as the regioisomer.

### Example 12

### Preparation of Taxol Derivative (MPA-EEA-Docetaxel)

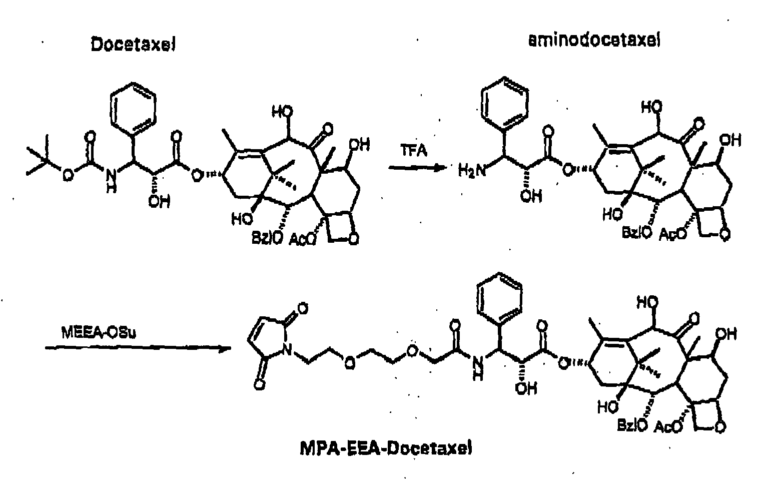

To prepare MPA-EEA-docetaxel, docetaxel is first converted to the free amino compound by treatment with trifluoroacetic acid in dichloromethane (Docetaxel (0.1 mmol) is treated with TFA (0.5 mL) for 10 min). TFA is evaporated and the residue, containing aminodocetaxel, is used for the next step.

N-Methylmorpholine is added to the THE solution (5 mL) containing aminodocetaxel (0.1 mmol) and succinyl 2-(2-mateimido)ethoxyethoxyacetate (succinate ester of MEEA)(0.2 mmol). The reaction is stirred for 30 min or until complete. The solvent is evaporated and the residue is purified by preparative HPLC to give MPA-EEA. docetaxel.

This process is not limited to this set of conditions one skilled in the art can also use conditions where the amino compound is dissolved In dichloromethane and treated with bis-(trimethylsilyl)-trifluoroacetamide to silylate the free alcohols. Concentrate and redissolve in DMF and add MPA-EEA-CO₂H and ethyl-dimethyl-aminopropylearbodlimide (EDC) or dicyclohexylcarbodiimde (DCC) and allow to react for 16 h. The resudue of this reaction is dissolved in methanol and a catalytic amount of TFA is added and the mixture is allowed to react for 2h, The reaction mixture is then concentrated and the residue is purified by HPLC to give MPA-EEA-docetaxel.

### Example 13

### Preparation of Taxol Derivative (C3'-NHS-glutamyl-docataxel)

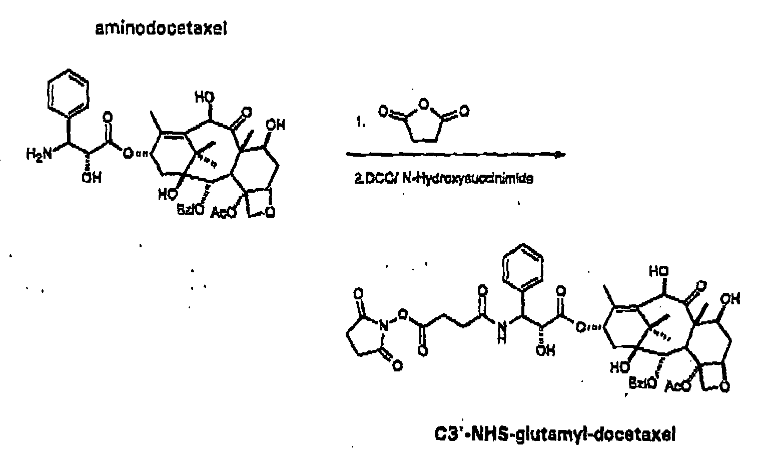

The aminodocetaxel (0.1 mmol) synthesized in Example 12 above is reacted with succinic anhydride (0.2 mmol) and N-methylmorpholine in THE (10 mL) and stirred for 60 min, or until complete. THF is evaporated and the residue extracted with EtOAc. The combined EtOAc layers is washed with water and dried (Na₂SO₄). The solvent is evaporated and the residue purified by flash column chromatography to give the succinic acid derivative.

The residue (0.1 mmol) obtained above is treated with DCC (0.12 mmol) and N-hydroxysuccinimide (10 mmol) for 60 min or until complete. The product is extracted with EtbAc and the extract is washed with water and dried (Na₂SO₄), The solvent is evaporated and the residue purified by preparative HPLC to give C3'-NHS-glutamyl-docetaxel.

### Example 14

### Preparation of Taxol Derivative (C3'-MPA-adipyl-docetaxel)

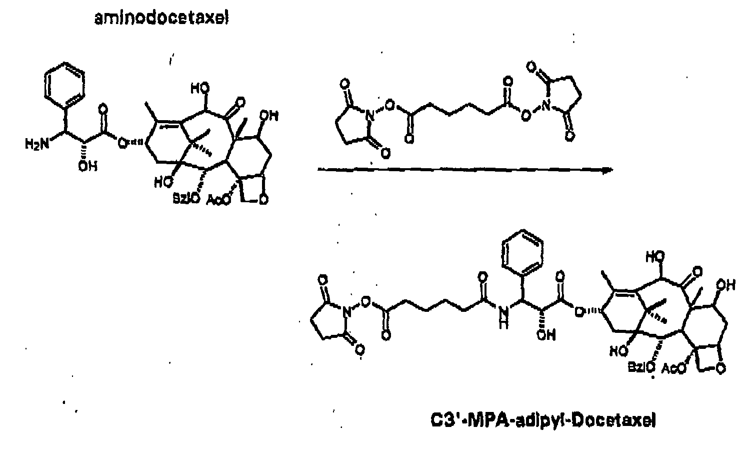

The aminodocetaxel (0,1 mmol) synthesized in Example 12 above is reacted with disuccinyl adipate (0.2 mmol) and N-methylmorpholine (0.4 mmol) in THF are stirred at room temperature for 60 min or until complete. THF is evaporated and the residue is purified by preparative HPLC to give C3'-MPA-adipyl-docetaxel.

### Example 15

### Preparation of Methotrexate Derivative (MPA-EEA-Methotrexate)

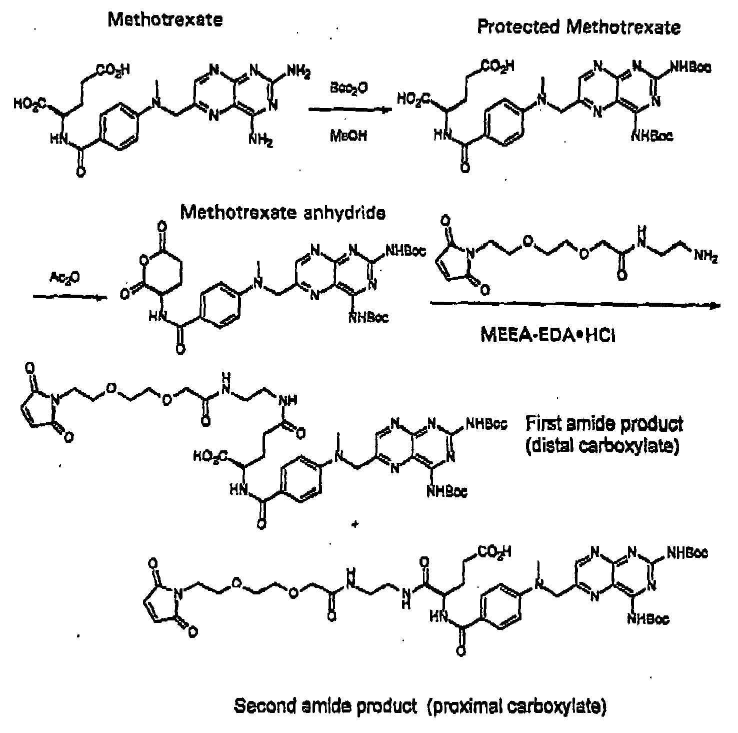

To synthesize MPA-EEA-Methotrexate, first the amine groups on methotrexate are protected as Boc derivates by reaction with di-t-butyloxy carbonyl anhydride in a suitable solvent, preferably methanol. Specifically, methotrexate (1 mmol) in methanol (10 mL) are treated with Boc₂O (4 mmol) for 30 min. Methanol is evaporated and the crude product is purified by flash column chromatography on silica gel or preparative HPLC to yield the protected methotrexate.

The protected methotrexate (1 mmol) is next treated with Ac₂O (10 mL) at 40 °C for 60 min. The excess Ac₂O is evaporated under reduced pressure. Ether is triturated to remove the remaining solvent and the product, methotrexate anhydride, is pumped dry. The crude product is used for the next step.

N-Methylmorpholine (2 mmol) are added to the methylene chloride solution (40 mL) containing the crude methotrexate anhydride (1 mmol) and MEEA-EDA.HCI (1.2 mmol). The reaction is stirred at room temperature for 30 min. The methylene chloride is washed with HCl (1N), water and dried (Na₂SO₄). This reaction leads to a mixture of amide products. The first amide product is a result of a reaction on the distal carboxylate and the the second amide product is a result of a reaction on the proximal carboxylate. The compounds are separated by chromatography and the individual compounds are then deprotected with TFA diluted in dichloromethane, concentrated and the residue purified by HPLC. Each of these compounds is treated with TFA, and the product is purified by preparative HPLC, resulting in deprotected products as shown below.

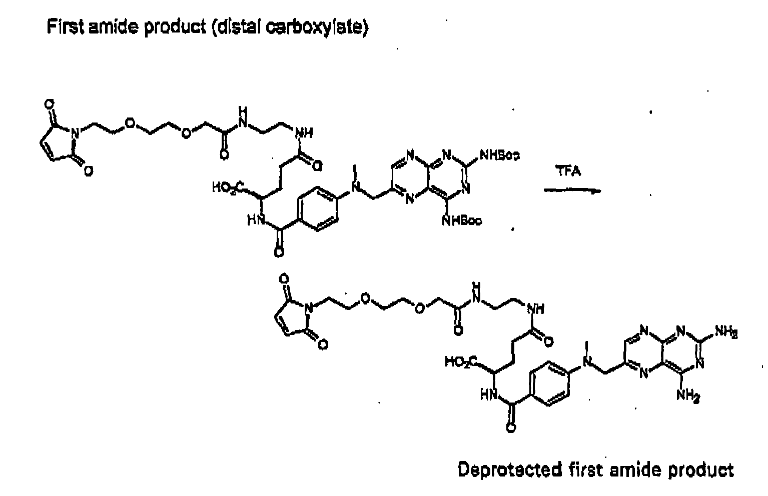

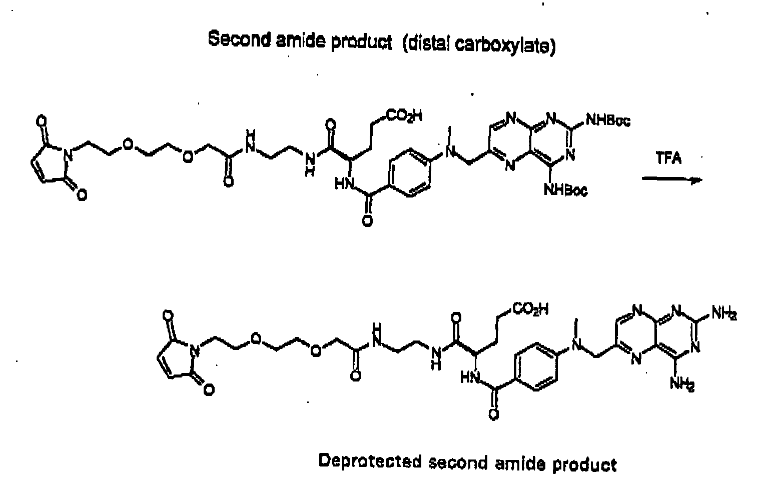

### Example 16

### Preparation of Methotrexate Derivative (NHS-Methotrexate.)

Initially, t-butyl-Methotrexate was prepared according to the procedure described by Rosowsky et al. J. Med. Chem., 1991, 34, 574-579. The preferred site of attachment of the linker is the position based on the SAR described in the above publication.

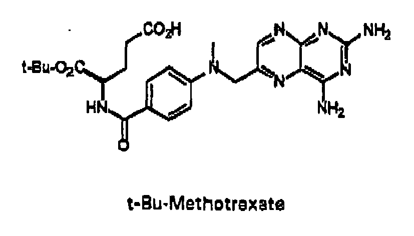

The t-Bu-methotrexate is then converted to the diBoc derivative using the di-*t*-butyloxycarbonyl anhydride, as described in more detail below. The methyl ester of caprioic acid is then coupled to the diBoc derivative using DCC as a coupling agent in the presence HOBT of at ambient temperature to give compound C. The methyl ester of compound C is then hydrolyzed using dilute sodium hydroxide in methanol and water and acidification to pH 4 then extraction with ethyl acetate, drying (Na₂SO₄) and concentration led a residue which is converted to the succinate ester by first forming the mixed anhydride with isobutyl chloroformate in the presence of NMM in dichloromethane. The residue is then deprotected using TFA diluted in dichloromethane and concentrated and the residue is purified by HPLC to give NHS-methotrexate.

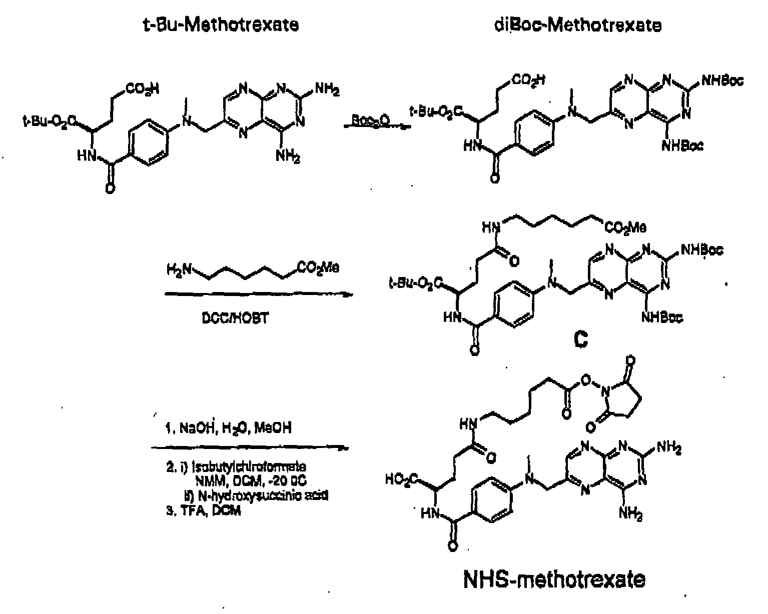

Described in more detail, the t-Bu-methotrexate (1 mmol) and Boc₂O (24 mmol) in MeOH (10 mL) are stirred for 60 min. or until complete. MeOH is evaporated and the residue purified by flash column chromatography to give diBoc-methotrexate. The diBoc-methotrexate (1 mmol), DCC (2 mmol) and HOBT (2 mmol) in methylene chloride 910 mL) are stirred at room temperature for 120 min. Then methyl 6-aminocaproate (2 mmol) is added. The reaction is stirred for 80 min or until complete. The reaction is quenched with AcOH (1 mL). Precipitate is removed by filtration. The filtrate is washed with water and dried (Na₂SO₄). The solvent is evaporated and the residue purified by flash column chromatography to give compound C.

The compound C (1 mmol) is treated with NaOH (1 N, 10 mL) for 30 min or until complete. The aqueous solution is adjusted to pH 4 by AcOH. The product is extracted with EtOAc. Th combined extracts are washed with water, dried (Na2SO4). The solvent is evaporated and the residue purified by flash column chromatography to give the carboxylic acid. The acid (1 mmol) is treated with isobutyl chloroformate (2 mmol) in the presence of N-mothylmorpholine in methylene chloride (20 mL) at -20 0C for 30 min. N-Hydroxysuccinimide (4 mmol) is added. The reaction is stirred at room temperature for 60 min. The methylene chloride solution is washed with HCl (1N), and dried. The solvent is evaporated and the residue purified by flash column chromatography. The product Is treated with TFA (5 mL) for 30 min. TFA is evaporated end the residue purified by preparative HPLC to give NHS-methotrexate.

### Example 17

### Preparation of Hydroxyurea Derivative (MPA-EEA-Hydroxyurea)

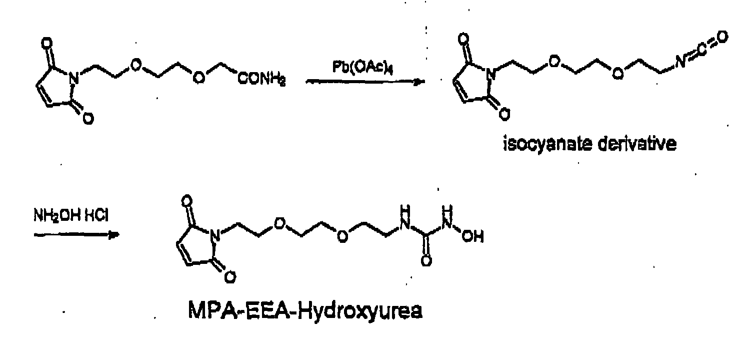

To prepare MPA-EEA-Hydroxyurea, first 2-(2-maleimido)ethoxyethoxyacetamide (10 mmol) in toluene (50 mL) is treated with lead tetraacetate (20 mmol) for 2 hours or until complete The reaction mixture is washed with water and dried (Na₂SO₄). Toluene is evaporated and the residue purified by flash column chromatography on silica get to give the isocyanate derivative. Next, the isocyanate derivative (5 mmol) cooled to -40°C with hydroxyamine hydrochloride (5 mmol) in methylene chloride (30 mL). To the reaction mixture N-methylmorpholine (5 mmol) is added slowly. The reaction is warmed up to room temperature. The methylene chloride solution is washed with HCl (1 N), water and dried (Na₂SO₄). The product, MPA-EEA-Hydroxyurea, is purified by flash column chromatography on silica gel.

### Example 18

### Preparation of Tamoxifen Derivative (MPA-Tamoxifen)

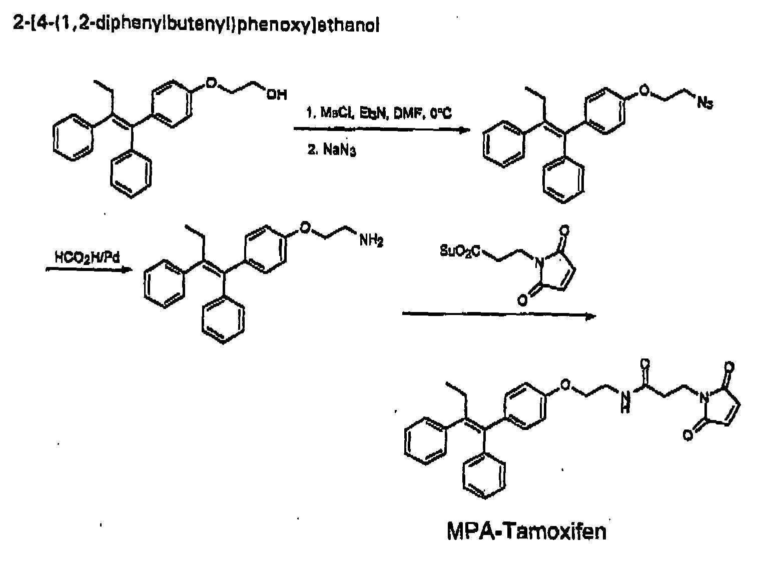

To prepare MPA-tamoxifen, mesyl chloride (12 mmol) is added to the DMF (20 mL) solution containing 2-[4-(1,2-diphenylbutenyl)phenoxy]ethanol (G. C.Crawley EP 19377 B1 April 4 1982) (10 mmol) and triethylamine (12 mmol) at 0°C. The reaction is stirred for 60 min or until complete. Then NaN₃ (30 mmol) is added and the reaction mixture is stirred at 50°C for 60 min. The solvent is evaporated under reduced pressure. The residue is purified by flash column chromatography to give 2-[4-(1,2-diphenylbutenyl)phenoxy]ethyl azide.

At reflux, formic acid (95%, 50 mmol) is added dropwise to the mathanol mixture of the tamoxifen azide (10 mmol) and Pd(OAc)₂ (0.5 mmol). The reaction is stirred at reflux for 30 min. The catalyst is removed by filtration and the solvent evaporated to dryness. The crude tamoxifen amine product is used for the next step.

Diisopropylethylamine (12 mmol) is added to the methylene chloride (30 mL) solution containing the tamoxifen amine (10 mmol) and succinyl 3-maleimidopropanoate (12 mmol). The reaction is stirred for 60 min or until complete. The methylele chloride solution is washed with HCI, water and dried. Evaporation of the solvent gives a residue, which is purified by flash column chromatography to yield MPA-tamoxifen.

### Example 19

### Preparation of Tamoxifen Derivative (NHS-adipyl-Tamoxifen)

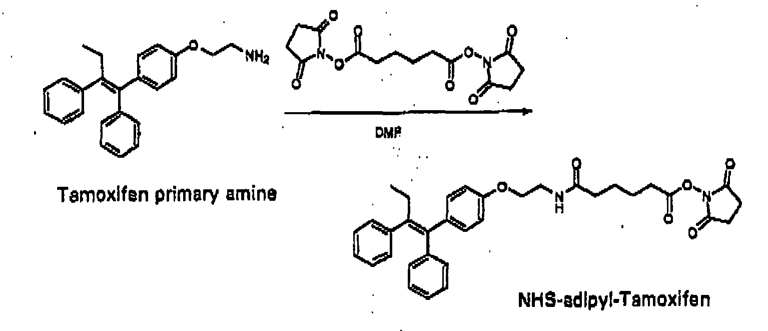

The tamoxifen primary amine (1 mmol) prepared above, along with disuccinyl adipate (2 mmol) and N-methylmorpholine (2 mmol) in methylene chloride (10 mL) are stirred for 60 min. or until the reaction Is complete. The methylene chloride is washed with water and dried. The solvent is evaporated and the residue purified by flash column chromatography on silica gel to give the six carbon chain N-hydroxysuccinate ester.

### Example 19

### Preparation of Mitomycin Derivative (N-1a-MPA-Mitomycin C)

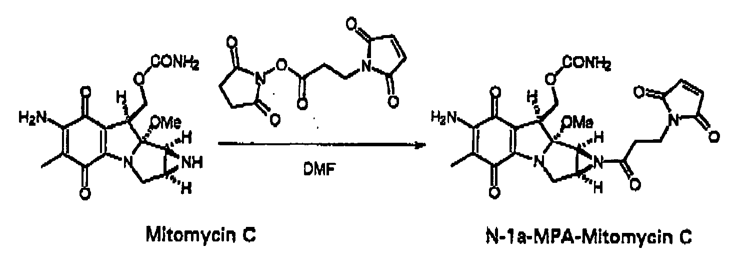

To prepare N-1a-MPA-mitomycin C, mitomycin C (1 mmol) is treated with succinyl 3-maleimidopropanoate (1.2 mmol) in methylene chloride (10 mL) for 16 hours or until complete. The methylene chloride is washed with water and dried (Na₂SO₄). The solvent is evaporated and the residue purified by preparative HPLC to give N-1a-MPA-Mitomycin C.

### Example 20

### Preparation of Mitomycin Derivative (N-1a-NHS-adipyl-Mitomycin C)

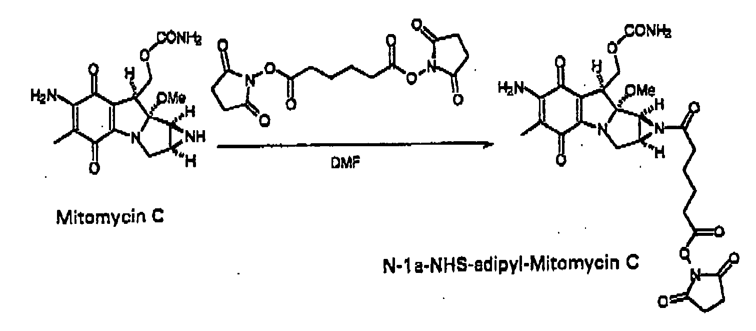

Mitomycin C is converted to the six carbon chain N-hydroxysuccinate ester by reaction with di-N-hydroxysuccinyl adipate in DMF using the following method:

Mitomycin C (1 mmol) is treated with disuccinyl adipate (1 mmol) in methylene chloride (10 mL) for 16 hours or until complete. The methylene chloride is washed with water and dried (Na₂SO₄). The solvent is evaporated and the residue purified by preparative HPLC to give N-1a-NHS-adipyl-Mitomycin C.

### Example 21

### Preparation of Vincristine derivative (MPA-Vincristine)

MPA-vincristine is prepared by attaching the reactive group to vincristine analogs at the C23 carbonyl or the C3 position on the ring using a hydrazide functionality described in the literature (B. C. Laguzza et al., US 4,801,688. January 31 1989, Culliman US 4,203,898 May 1980).

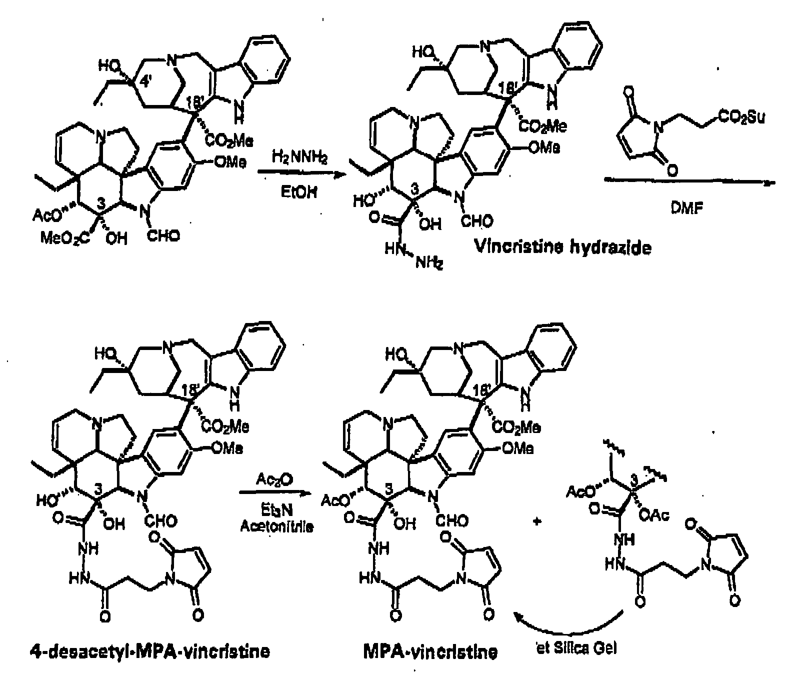

More specifically, vincristine (25 mg, 0.027 mmol) or alternatively, vinblastin (with a methyl group instead of a formyl at the N, position), is dissolved in ethanol (0.5 ml) and treated with anhydrous hydrazine (0.2 mmol). The mixture is heated at 60°C in a sealed tube for 4h. The reaction mixture is concentrated under reduced pressure and vacuum, and the residue is purified by flash column chromatography to give vincristine hydrazide. Vincristine hydrazide (0,01 mmol) is dissolved in DMF (0.5 ml) and a solution of succinyl 3-maleimidopropanoate (0.015 mmol) in DMF (0.1 ml) is added at ambient temperature and the reaction mixture is stirred at that temperature for 16 h. The reaction mixture is concentrated under vacuum and the residue is purified by HPLC using the conditions previously described to give 4-desacetyl-MPA-vincristine.

Next, 4-desacetyl-MPA-vincristine (0.005 mmol) in methylene chloride (0.3 ml) is treated with acetic anhydride (20 µl of a 0.5 M solution in methylene chloride) and triethylamine (20 µl of a 0.5 M solution in methylene chloride) at 0°C. The mixture is stirred at ambient temperature for 2 h. The reaction mixture is diluted with methylene chloride and washed with saturated bicarbonate and brine, dried (Na₂SO₄) and concentrated. The residue is purified by HPLC to give MPA-vincristine and the C3 acetyl MPA-vincristine which can be converted to MPA-vincristine in the presence of wet silica (Hargrove US 3,392,173, July 1968).

### Example 22

### In vivo Half-life

The *in vivo* half-life of an antineoplastic agent derivative according to the invention can be determined by the *in vivo* administration of an antineoplastic agent derivative and investigation of the binding of the derivative to endogenous proteins *in vivo.* Specifically, rabbits would be injected with a solution of an antineoplastic agent derivative according to the invention. Blood samples would be taken and analyzed for the presence of the reactive group (for example maleimide) using gel electrophoresis. The same procedure would be performed with non-derivatized antineoplastic agents to serve as the control.

Alternatively, radioactively labeled antineoplastic agent derivatives (for example, ¹²⁵I labeled antineoplastic agent derivatives) may be injected into the test and the control groups. Animals would receive by slow injection (5 minutes) 50 µg/Kg of conjugate diluted in glucose 5% with 1% of human serum albumin. One day after the injection, animals would receive 10 µcurie of ¹²⁵I-labelled antineoplastic agent derivative (100 µg per animal). One hour after the antineoplastic agent derivative injection, animals would be sacrificed, and various blood and tissue specimens would be collected. Control animals would receive the ¹²⁵labelled *underivatized* antineoplastic agent. Specific radioactivity would be measured using a gamma counter and expressed in cpm per ml of blood or cpm per gram of tissue.

### Example 23

### Binding of NHS-Antineoplastic Agent to RBCs and Plasma Proteins

To measure the binding of an NHS-antineoplastic agent to RBC and plasma proteins, 5 mg (-1.5 mg/kg) and 50 mg (-15 mg/kg) of an NHS-antineoplastic agent, for example NHS-C8-doxorubucin, C3'-NHS-glutamyl-docetaxel, NHS-methotrexate, N-1-a-NHS-adipyl-MitomycinC, or NHS-adipyl-tamoxifensotubilized in DMSO may be injected into rabbits. Blood samples would be then taken at 0.5, 1, 2 and 4 h on the same day after injection and then on, for example, 1, 2, 3, 6, 9, 13, 20, 27, 34, 41, 48 and 55 days after injection. The binding of the NHS-antineoplastic agent to RBCs would be shown by flow cytometry. The binding of the NHS-antineoplastic agent to plasma proteins would be analyzed by immunoblotting. The plasma proteins would be separated under reducing conditions (10 mM DTT) in a 10% polyacrylamide gel (Coomassie blue staining).

### Example 24

### Binding of MPA-Antineoplastic Agent to RBCs and Plasma Proteins

To measure the binding of an MPA-antineoplasticagent to RBC and plasma proteins, 5 mg (-1.5 mg/kg) and 50 mg (-15 mg/kg) of an MPA-antineoplasticagent, for example MPA-Doxorubicin, MPA-AEEA-Doxorubicin, MPA-AEE-Taxol, MPA-Taxol, MPA-EEA-Docetaxel, MPA-EEA-Methotrexate, MPA-EEA-Hydroxyurea, MPA-Tamoxifen, N-1 a-MPA-Mitomycin C, or MPA-Vnctristinesolubilized in DMSO may be injected into rabbits. Blood samples would be then taken at 0.5,1, 2 and 4 h on the same day after injection and then on 1, 2, 3, 6, 9, 13, 20, 27, 34, 41, 48 and 55 days after injection. The binding of the MPA-antineoplasticagent to RBCs would be shown by flow cytometry, The binding of the MPA-antineoplastic agent to plasma proteins would be analyzed by immunoblotting. The plasma proteins would be separated under reducing conditions (10 mM DTT) in a 10% polyacrylamide gel (Coomassie blue staining).

### Example 25

### Antitumor Effect of Doxorubicin Derivatives

The antitumor effect of doxorubicin derivatives according to the invention may be determined by comparing the antitumor effect of doxorubicin in mice with doxorubicin derivatives according to the invention. Primary kidney tumors would be induced by subcapsular injection of renal carcinoma cells in the mouse kidney. Doxorubicin as well as doxorubicin derivatives according to the invention would then be administered, and their antitumor effects compared. Sample dosages that may be used would include: 4 x 10 mmol/kg for doxorubicin (equal to 4 x 6 mg/kg, Mᵣ = 580.0 g/mol) and 4 x 20 mmol/kg for the doxorubicin derivative (equal to 4 x 16.7 mg/kg, Mᵣ = 807.8 g/mol). After 24 hours, body weight loss in both treated groups would be measured to determine the efficacy of the doxorubicin derivatives as compared to underivatized doxorubicin.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. Use of a composition for the manufacture of a medicament, said composition comprising a derivative of an antineoplastic agent and analogs thereof wherein the derivative includes a reactive group which reacts with amino groups, hydroxyl groups, or thiol groups on blood components to form stable covalent bonds, said reactive group being selected from N-hydroxysuccinimide, *N*-hydroxy-sulfosuccinimide and a maleimide group for use in the treatment of cancer.

2. Use of a composition according to Claim 1 wherein the antineoplastic agent is selected from the group consisting of doxorubicins, taxols, methotrexates, hydroxyurea, tamoxifen, mytomycin C, platinum complexes, vinca alkaloids, and analogs thereof.

3. Use of a composition according to Claim 2 wherein the antineoplastic agent is selected from doxorubicins and analogs thereof.

4. Use of a composition according to Claim 2 wherein the antineoplastic agent is selected from taxols and analogs thereof.

5. Use of a composition according to Claim 2 wherein the antineoplastic agent is selected from methotrexates and analogs thereof.

6. Use of a composition according to Claim 2 wherein the antineoplastic agent is selected from hydroxyurea and analogs thereof.

7. Use of a composition according to Claim 2 wherein the antineoplastic agent is selected from tamoxifen and analogs thereof.

8. Use of a composition according to Claim 2 wherein the antineoplastic agent is selected from mytomycin C and analogs thereof.

9. Use of a compostion according to Claim 1 wherein the derivative is reactive with blood proteins.

10. Use of the composition according to Claim 1 wherein the derivative is reactive with a thiol group on a blood protein.

11. Use of the composition according to Claim 1 wherein the derivative is reactive with albumin to form a stable covalent bond.

12. Use of the composition according to Claim 1 wherein the reactive group is N-hydroxysuccinimide or N-hydroxysulfosuccinimide.

13. Use of the composition according to Claim 1 wherein the reactive group is a maleimide group.

14. A method for extending the lifetime of a antineoplastic agent *in vivo,* comprising:
(a) modifying said antineoplastic agent by attaching a reactive group to a site sufficiently far away from the pharmacophore region such that the antineoplastic agent derivative substantially retains its therapeutic activity and is capable of forming a covalent bond with a functionality on a blood component;
(b) administering said agent to a host; and
(c) forming a covalent bond between said reactive group and a functionality on a blood component *in vivo* to form a antineoplastic agent derivative-blood component conjugate, thereby extending the lifetime of the antineoplastic agent.

15. A method according to claim 14, further comprising the step of administering said antineoplastic agent derivative *in vivo* before step (b), such that the antineoplastic agent derivative-blood component conjugate is formed *in vivo.*

16. A method according to claim 14, wherein said reactive group is a maleimido group.

17. A method according to claim 14, wherein said reactive group is attached to said antineoplastic agent via a linking group.

18. A method according to claim 17, wherein said linking group is -CH₂-CH₃-CH₃-CH₃-CH₃-CH₂-.

19. A method according to claim 17, wherein said linking group is -CH₂-.

20. A method according to claim 17, wherein said linking group is -O-CH₂-CH₂-O-CH₂-CH₂- or -(O-CH₂-CH₂O)₂-CH₂-CH₂-.

21. A method according to claim 14, wherein said blood component is albumin.

22. A antineoplastic agent derivative according to claim 14, wherein the antineoplastic agent is a compound of formula: 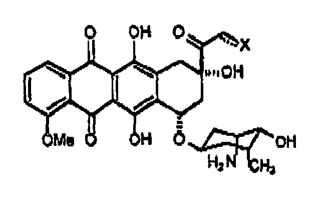 wherein X is selected from the group consisting of ether, thioether, secondary amine, tertiary amine, secondary amide, tertiary amide, eater, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate.

23. A method according to claim 22, wherein X is a primary amide.

24. A antineoplastic agent derivative according to claim 14, wherein the antineoplastic agent is a compound of formula: 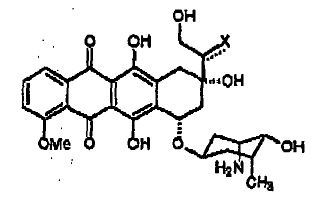 wherein X is selected from the group consisting of ether, thioether, secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate.

25. A method according to claim 24, wherein X is a primary amide.

26. A antineoplastic agent derivative according to claim 14, wherein the antineoplastic agent is a compound of formula: 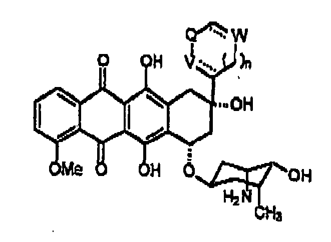 wherein:
V is O, N, S, or C;
W is O, N, S, or C;
n is 0 or 1; and
Q is an oxygen, sulfur, secondary or tertiary amine, secondary or tertiary amide, oxygenated phophorus, or a substituted or unsubstituted carbon atom.

27. A method according to claim 26, wherein V is an oxygen, Q is a nullity and W is a primary amine.

28. A antineoplastic agent derivative according to claim 14, wherein the antineoplastic agent is a compound of formula: 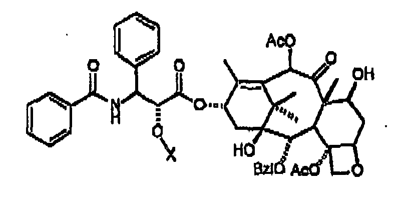 wherein X is selected from the group consisting of ether, thioether, secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate.

29. A method according to claim 28, wherein X is a -NH- or -CO-.

30. A antineoplastic agent derivative according to claim 14, wherein the antineoplastic agent is a compound of formula: 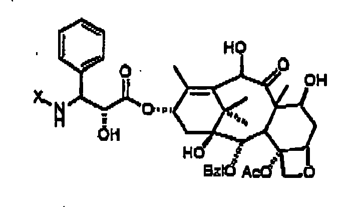 wherein X is selected from the group consisting of ether, thioether, secondary amine, tertiary amine, secondary amide, tertiary amide, ester, thioester, imine, hydrazone, semicarbazone, acetal, hemi-acetal, ketal, hemi-ketal, aminal, heml-aminal, sulfonate, sulphate, sulfonamide, sulfonamidate, phosphate, phopharamide, phosphonate, and phosphonamidate.

31. A method according to claim 30, wherein X is -CO-.

32. A antineoplastic agent derivative according to claim 14, wherein the antineoplastic agent is a compound of formula: 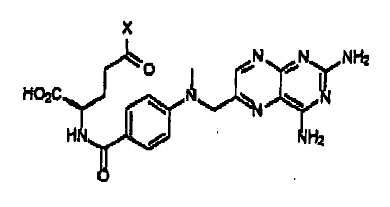 wherein X is selected from the group consisting of ether, thioether, secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate.

33. A method according to claim 32, wherein X is -NH-.

34. A antineoplastic agent derivative according to claim 14, wherein the antineoplastic agent is a compound of formula: 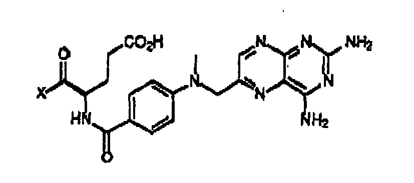 wherein X is selected from the group consisting of ether, thioether, secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate.

35. A method according to claim 34, wherein X is -NH-.

36. A antineoplastic agent derivative according to claim 14, wherein the antineoplastic agent is a compound of formula: 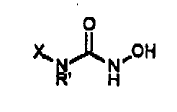 wherein X is selected from the group consisting of ether, thioether, secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate; and
R' is a hydrogen, alkyl, aryl, or acyl.

37. A method according to claim 36, wherein X is -CO-.

38. A antineoplastic agent derivative according to claim 14, wherein the antineoplastic agent is a compound of formula: 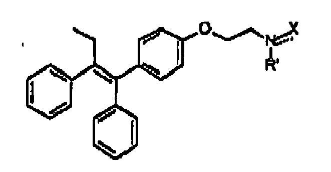 wherein X is selected from the group consisting of ether, thioether, secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate; and
R' is a hydrogen, alkyl, aryl, or acyl,

39. A method according to claim 38, wherein X is -NH-CO-,

40. A antineoplastic agent derivative according to claim 14, wherein the antineoplastic agent is a compound of formula: 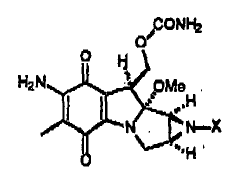 wherein X is selected from the group consisting of ether, thioether, secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate.

41. A method according to claim 40, wherein X is -CO-.

42. A antineoplastic agent derivative according to claim 14, wherein the antineoplastic agent is a mixture of four regioisomers of formulae: 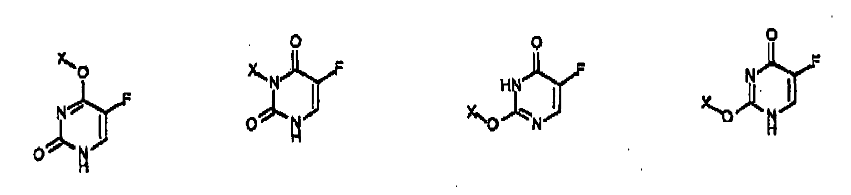 wherein X is selected from the group consisting of other, thioether, secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate.

43. A method according to claim 42, wherein X is -CO-.

44. A antineoplastic agent derivative according to claim 14, wherein the antineoplastic agent is a compound of formula: 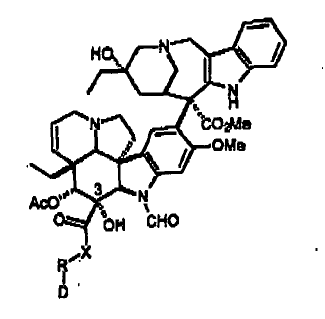 wherein X is selected from the group consisting of secondary amine, tertiary amine, secondary amide, tertiary amide, ester, thioester, imine, hydrazone, semicarbazone, acetal, hemi-acetal, ketal, hemi-ketal, aminal, hemi-aminal, sulfonate, sulphate, sulfonamide, sulfonamidate, phosphate, phophoramide, phosphonate, and phosphonamidate.

45. A composition comprising a compound of the formula:

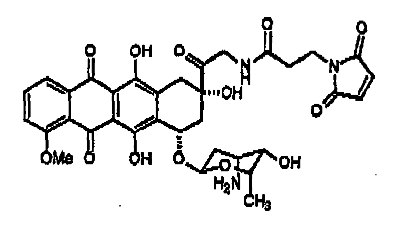

46. Use of a composition for the manufacture of a medicament for use in the treatment of cancer, said composition comprising a compound of formula:

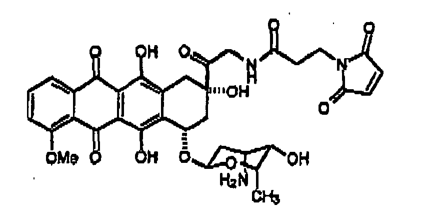

47. A composition comprising a compound of the formula:

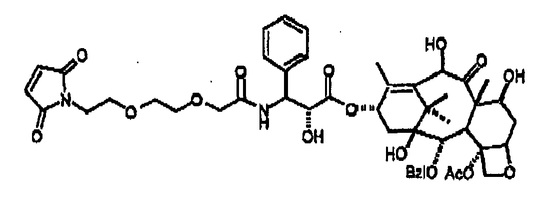

48. Use of a composition for the manufacture of a medicament for use in the treatment of cancer, said composition comprising a compound of formula:

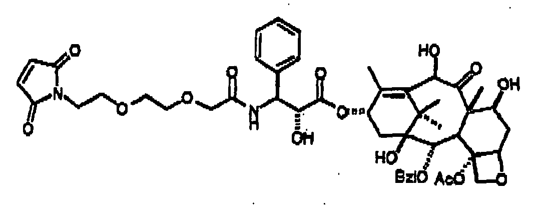

49. A composition comprising a compound of the formula:

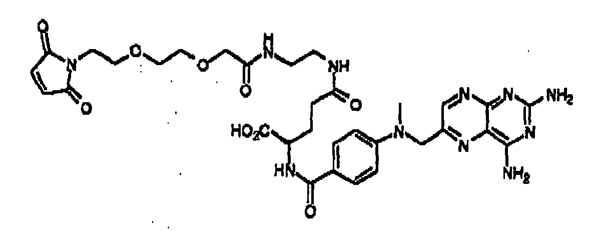

50. Use of a composition for the manufacture of a medicament for use in the treatment of cancer, said composition comprising a compound of formula:

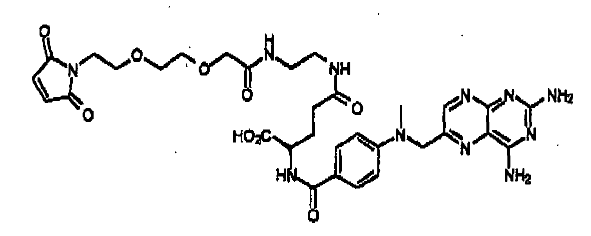

51. A composition comprising a compound of the formula:

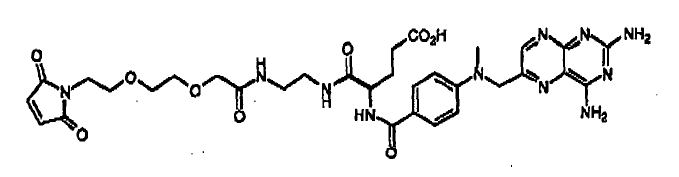

52. Use of a composition for the manufacture of a medicament for use in the treatment of cancer, said composition comprising a compound of formula:

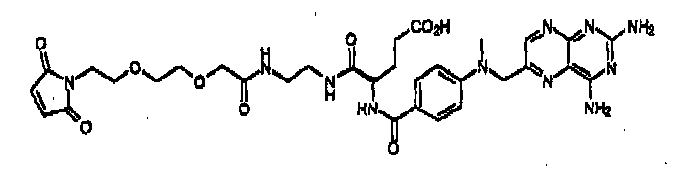

53. A composition comprising a compound of the formula:

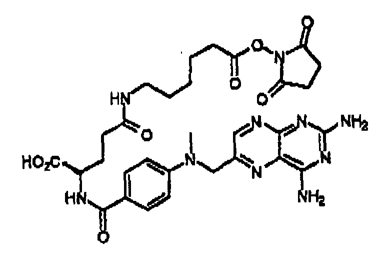

54. Use of a composition for the manufacture of a medicament for use in the treatment of cancer, said composition comprising a compound of formula:

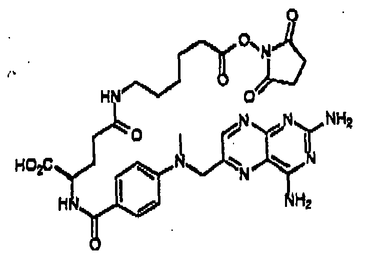

55. A composition comprising a compound of the formula:

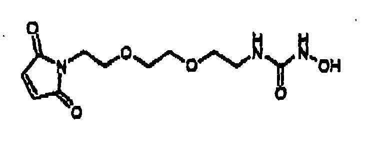

56. Use of a composition for the manufacture of a medicament for use in the treatment of cancer, said composition comprising a compound of formula:

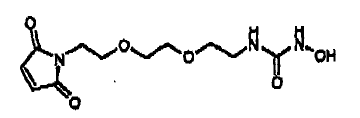

57. A composition comprising a compound of the formula:

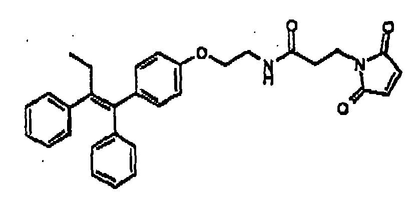

58. Use of a composition for the manufacture of a medicament for use in the treatment of cancer, said composition comprising a compound of formula:

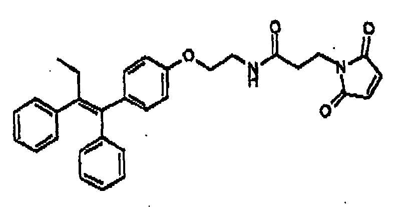

59. A composition comprising a compound of the formula:

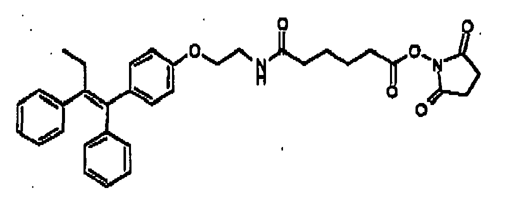

60. Use of a composition for the manufacture of a medicament for use in the treatment of cancer, said composition comprising a compound of formula:

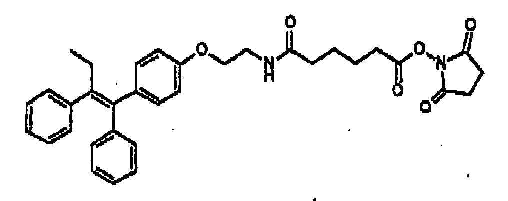

61. A composition comprising a compound of the formula:

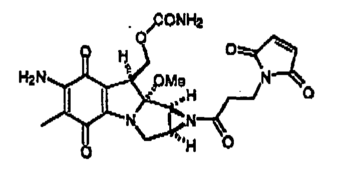

62. Use of a composition for the manufacture of a medicament for use in the treatment of cancer, said composition comprising a compound of formula:

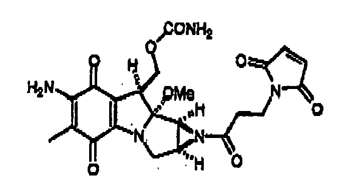

63. A composition comprising a compound of the formula:

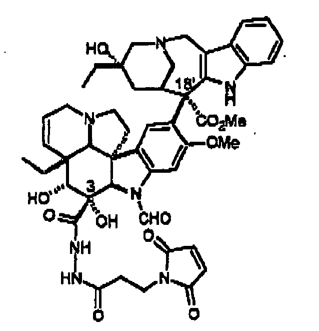

64. Use of a composition for the manufacture of a medicament for use in the treatment of cancer, said composition comprising a compound of formula:
